(19) Europäisches Patentamt
European Patent Office
Office européen des brevets

(11) **EP 4 628 064 A1**

(12) **EUROPEAN PATENT APPLICATION**
published in accordance with Art. 153(4) EPC

(43) Date of publication:
**08.10.2025 Bulletin 2025/41**

(21) Application number: **23897345.7**

(22) Date of filing: **30.10.2023**

(51) International Patent Classification (IPC):
**A61H 7/00** *(2006.01)*   **A61H 23/00** *(2006.01)*
**A61F 7/00** *(2006.01)*

(52) Cooperative Patent Classification (CPC):
**A61H 23/02; A61F 7/00; A61H 7/00; A61H 7/001;**
**A61H 23/00;** A61H 2201/0207; A61H 2201/165;
A61H 2205/022; A61H 2230/00

(86) International application number:
**PCT/JP2023/039025**

(87) International publication number:
**WO 2024/116689 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.11.2022 JP 2022190806**

(71) Applicant: **Panasonic Intellectual Property
Management Co., Ltd.
Kadoma-shi, Osaka 571-0057 (JP)**

(72) Inventors:
• **KUSUKAME, Haruka**
  **Kadoma-shi, Osaka 571-0057 (JP)**
• **KAWASHIMA, Tomoko**
  **Kadoma-shi, Osaka 571-0057 (JP)**
• **ETO, Hiroki**
  **Kadoma-shi, Osaka 571-0057 (JP)**

(74) Representative: **Grünecker Patent- und
Rechtsanwälte
PartG mbB
Leopoldstraße 4
80802 München (DE)**

(54) **SKIN-CONTACT OR WEARABLE CARE DEVICE**

(57)    The present disclosure provides a care device with improved pleasantness when the care device is brought into contact with and worn on the skin. Care device (100) of the present disclosure is a care device that is brought into contact with or worn on the skin. Care device (100) of the present disclosure includes skin wearing member (1) containing an elastomer. Skin wearing member (1) has an Asker C hardness of 30 degrees or less and a coefficient of static friction of 3 or less. Here, the coefficient of static friction is a coefficient of static friction for an artificial skin sheet that satisfies the following characteristics (A) and (B). (A)A compressive stress of the artificial skin sheet at a time of being pushed in a thickness direction at a depth of 2 mm is between 0.01 MPa and 0.05 MPa inclusive. (B)Calculated average roughness Ra of a surface of the artificial skin sheet is from 5 $\mu$m to 10 $\mu$m.

**FIG. 1**

100

**Description**

TECHNICAL FIELD

The present disclosure relates to a care device that is brought into contact with or worn on the skin.

BACKGROUND ART

Many massage devices have been sold as devices for improving shoulder stiffness, eye fatigue, or hand and leg fatigue. In addition, many beauty treatment devices for beauty such as a beautiful face have been sold.

There has been an increasing need for "relaxation" and "soothing" in a stressed society. Care for stimulating a tactile sensation such as beauty treatment or massage can be mentioned as one of methods for obtaining relaxation and soothing. According to such care, it is considered that a user feels pleasant by being touched to the skin or being given a physical stimulation, or psychological and physiological good effects are obtained. In recent years, beauty treatment devices and massage devices for experiencing beauty treatment or massage at home have been developed.

PTL 1 proposes an eye mask for eliminating eye fatigue. In the eye mask, a Peltier element is attached to an annular frame positioned around the eye in the frame, a detachable pad is provided on an end surface on an eye side of the Peltier element, and a rectangular container filled with a heat storage agent is provided on the other end surface. The eye mask has a configuration in which a magnitude and an orientation of a current flowing through the Peltier element are controlled by a control device.

PTL 2 proposes a beauty device for eye care in which a facial skin around an eye is heated by water vapor and heat of a heating element.

PTL 3 proposes a footwear foot care pad containing an elastomer having a hardness of 20° to 40°, which can be detachably attached to an upper surface of an insole, for the purpose of treating pain of the foot and preventing fatigue.

Citation List

Patent Literature

PTL 1: Unexamined Japanese Patent Publication No. H07-16255
PTL 2: International Publication No. WO 2014/119025
PTL 3: Unexamined Japanese Patent Publication No. 2004-194734

Non-Patent Literature

NPL 1: "Age-Related Changes of the Three-Dimensional Structure in the Cheek Region", Koji Mizukoshi et al., Journal of Society of Cosmetic Chemists of Japan Vol. 43, No. 3 2009 177-183

SUMMARY OF THE INVENTION

Technical problem

Many care devices such as massage devices and beauty treatment devices are directly brought into contact with or worn on the skin. Accordingly, in these care devices, touch pleasantness when the care device is directly brought into contact with the skin and wearing pleasantness when the care device is worn are important for obtaining relaxation and soothing effects. However, in these care devices of the related art, the pleasantness when the care device is brought into contact with the skin and when the care device is worn is not sufficient, and sufficient relaxation and soothing effects have not been obtained.

The present disclosure provides a care device with improved pleasantness when the care device is brought into contact with and worn on the skin.

Solution to problem

The present disclosure provides a care device that is brought into contact with or worn on a skin, including a skin wearing member containing an elastomer,
in which the skin wearing member has an Asker C hardness of 30 degrees or less and a coefficient of static friction of 3 or less.

Here, the coefficient of static friction is a coefficient of static friction for an artificial skin sheet that satisfies the following characteristics (A) and (B).

(A) A compressive stress of the artificial skin sheet at a time of being pushed in a thickness direction at a depth of 2 mm is between 0.01 MPa and 0.05 MPa inclusive.
(B) Calculated average roughness Ra of a surface of the artificial skin sheet is from 5 $\mu$m to 10 $\mu$m.

Advantageous effect of invention

The present disclosure provides a care device with improved pleasantness when the care device is brought into contact with and worn on the skin.

BRIEF DESCRIPTION OF THE DRAWINGS

Fig. 1 is a cross-sectional view illustrating an example of a care device according to a first exemplary embodiment.
Fig. 2 is a cross-sectional view illustrating an example of a skin wearing member having a multilayer structure.
Fig. 3A is a cross-sectional view illustrating a configuration in which a base member is sealed with a film in the skin wearing member.
Fig. 3B is a cross-sectional view illustrating a configuration in which the base member is sealed with the film in the skin wearing member.
Fig. 4 is a cross-sectional view illustrating a first modification of the care device according to the first exemplary embodiment.
Fig. 5 is a cross-sectional view illustrating a second modification of the care device according to the first exemplary embodiment.
Fig. 6 is a cross-sectional view illustrating a third modification of the care device according to the first exemplary embodiment.
Fig. 7 is a cross-sectional view illustrating a fourth modification of the care device according to the first exemplary embodiment.
Fig. 8 is a cross-sectional view illustrating a fifth modification of the care device according to the first exemplary embodiment.
Fig. 9 is a cross-sectional view illustrating a sixth modification of the care device according to the first exemplary embodiment.
Fig. 10 is a graph representing a relationship between Asker C hardness of the skin wearing member and pleasantness of a touch feeling of the eye and hand when the care device is used.
Fig. 11 is a graph representing a relationship between a coefficient of static friction of the skin wearing member for an artificial skin and pleasantness of the touch feeling of the eye and hand when the care device is used.

DESCRIPTION OF EMBODIMENT

(Underlying knowledge and the like of present disclosure)

At the time when the present inventors have arrived at the present disclosure, the importance of improving a touch feeling of a portion that comes into contact with the skin in a care device that is brought into contact with or worn on the skin has not been recognized so much. However, the present inventors have found that improvement of the touch feeling of the portion that comes into contact with the skin in the care device is effective for relaxation and soothing effects of the care device, and have intensively studied improvement of the touch feeling of the portion that comes into contact with the skin in the care device.

It is considered that a physical property value of an object affects a touch feeling when a person touches the object. The present inventors have newly found that, when the person touches the object, the touch feeling felt by the person correlates with hardness of the object and a coefficient of static friction for the skin of the object, and the touch feeling can be controlled by the hardness and the coefficient of static friction of the object. The person feels softness when the hardness of the object is low. As a result of intensive research, the present inventors have found that, when an Asker C hardness of the object is less than or equal to 30 degrees, the pleasantness of the touch feeling to the skin is improved. In addition, as described above, regarding the coefficient of static friction of the object, the coefficient of static friction for the skin of the person correlates with the touch feeling. Thus, in order to represent the touch feeling of the object for the skin of the person, a coefficient of friction for the skin of the person which is the body to be rubbed is required. However, it has been difficult to evaluate the coefficient of friction for the skin of the person having unique surface characteristics and softness. The present inventors have found that a coefficient of static friction for an artificial skin close to the physical property of the skin of the person serves as an index of the touch feeling for the skin of the person.

As a result of intensive studies, the present inventors have found that, in a case where the coefficient of static friction of the object for the artificial skin, which is a material close to the skin of the person, satisfies 3 or less, the pleasantness of the touch feeling to the skin is improved.

The artificial skin is a product that imitates the skin of the person and brings a touch feeling, softness, and the like close to the skin of the person, and is used for suture training in a medical field, evaluation of cosmetics, and the like. The present inventors have focused on specific surface characteristics and softness of the skin of the person, clarified surface characteristics and physical properties as an index of softness, and found that the coefficient of static friction for the skin of the person can be evaluated by using the artificial skin having the physical property close to the skin of the person.

Therefore, the present disclosure provides a care device that is brought into contact with or worn on a skin, including a skin wearing member containing an elastomer, in which the skin wearing member has an Asker C hardness of 30 degrees or less and a coefficient of static friction of 3 or less. Note that, in the present disclosure, the Asker C hardness and the coefficient of static friction of the skin wearing member are an Asker C hardness and a coefficient of static friction of a surface of the skin wearing member coming into contact with the skin when the care device is brought into contact with or worn on the skin. Here, the coefficient of static friction is a coefficient of static friction for an artificial skin sheet that satisfies the following characteristics (A) and (B).

(A) A compressive stress of the artificial skin sheet at a time of being pushed in a thickness direction at a depth of 2 mm is between 0.01 MPa and 0.05 MPa inclusive.
(B) Calculated average roughness Ra of a surface of the artificial skin sheet is from 5 $\mu$m to 10 $\mu$m. That is, calculated average roughness Ra of the surface of the artificial skin sheet is between 5 $\mu$m and 10 $\mu$m inclusive.

Note that, in the present disclosure, the Asker C hardness of the skin wearing member is a value at a room temperature (for example, 25°C). In addition, a compressive stress of the artificial skin sheet to be used at the time of being pushed in a thickness direction at a depth of 2 mm is also a value at a room temperature (for example, 25°C).

Hereinafter, an exemplary embodiment will be described in detail with reference to the drawings. However, unnecessary detailed descriptions may be omitted. For example, detailed descriptions of already well-known matters or redundant descriptions of substantially the same configuration may be omitted. This is to avoid unnecessary redundancy in the following description and to facilitate understanding by those skilled in the art.

Note that, the accompanying drawings and the following descriptions are only provided to facilitate a person skilled in the art to fully understand the present disclosure, and are not intended to limit the subject matters as defined in the claims.

(First exemplary embodiment)

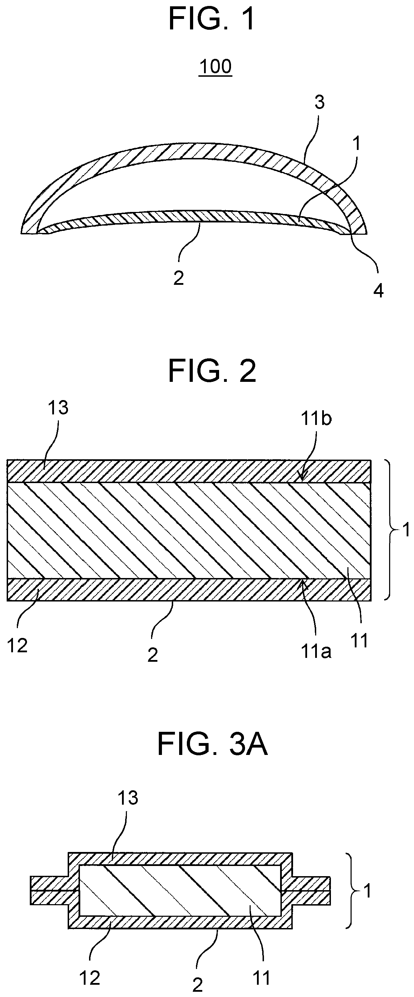

Hereinafter, an exemplary embodiment will be described with reference to Figs. 1 to 8.

[1-1. Configuration]

A care device according to a first exemplary embodiment is a care device to be brought into contact with or worn on the skin. The care device according to the present exemplary embodiment includes a skin wearing member containing an elastomer. The skin wearing member has an Asker C hardness of 30 or less and a coefficient of static friction of 3 or less. Note that, in the present exemplary embodiment, the Asker C hardness and the coefficient of static friction of the skin wearing member are an Asker C hardness and a coefficient of static friction of a surface of the skin wearing member coming into contact with the skin when the care device is brought into contact with or worn on the skin. Here, the coefficient of static friction is a coefficient of static friction for an artificial skin sheet that satisfies the following characteristics (A) and (B).

(A) A compressive stress of the artificial skin sheet at the time of being pushed in a thickness direction at a depth of 2 mm is between 0.01 MPa and 0.05 MPa inclusive.
(B) Calculated average roughness Ra of a surface of the artificial skin sheet is from 5 $\mu$m to 10 $\mu$m.

For example, "BIOSKIN BPS-01L" manufactured by Regina Fashion Supply Co., Ltd. can be used as the artificial skin sheet having the above-mentioned properties.

The care device according to the present exemplary embodiment includes the skin wearing member having the above configuration, and thus, the pleasantness when the skin wearing member is brought into contact with the skin and when the skin wearing member is worn on the skin can be improved.

In the present exemplary embodiment, the characteristics (A) and (B) of the artificial skin sheet used for specifying the coefficient of static friction of the skin wearing member can be obtained by the method described below.

The skin of the person has a multilayer structure including epidermis, dermis, subcutaneous tissue, and the like, and is a stacked body of layers having different elastic moduli. A thickness of the skin is about 2 mm on average. Softness of the stacked body can be evaluated by stress and strain with respect to a pushing force of a surface of the stacked body. As a result, the softness of the skin of the person was evaluated from the strain and stress when a pushing force was applied to the skin surface. In the present disclosure, a compressive stress obtained from a repulsive force when a pushing force having a depth of 2 mm in the thickness direction is applied to the surface of the artificial skin sheet is used as an index of the softness of the skin of the person.

The compressive stress at the time of being pushed to a depth of 2 mm was obtained by measuring a load when a sample (for example, skin of the person) was pressed by 2 mm at a speed of 10 mm/min against a surface of the sample by using, for example, force gauge systems ZTA-50N and MX2-500N-FA (manufactured by IMADA Co., Ltd.) and a probe having a cylindrical shape and having a diameter of 5 mm in a room temperature condition. For the compressive stress at the time of being pushed to 2 mm against the skin of the person, five points of each of the palm and the skin of the upper arm were measured.

A compressive stress of the skin of the person at the time of being pushed to 2 mm was in a range from 0.01 MPa to 0.05 MPa, inclusive. The same measurement was performed for "BIOSKIN BPS-01L" manufactured by Regina Fashion Supply Co., Ltd., which is one of artificial skins. As a result, the compressive stress at the time of being pushed to a depth of 2 mm was 0.026 MPa. As a result, it can be confirmed that a physical property value of "BIOSKIN BPS-01L" manufactured by Regina Fashion Supply Co., Ltd. is within the range of the compressive stress at the time of being pushed to 2 mm against the skin of the person and is close to the skin of the person.

The surface of the skin of the person has an irregularity shape based on wrinkles, pores, or the like. Surface characteristics due to the irregularity shape can be expressed by surface roughness. For the surface roughness of the skin of the person, NPL 1 discloses that calculated average roughness Ra of the skin of the cheek of a woman in her 20s to 50s is in a range from 5 μm to 16 μm, inclusive. The surface roughness of "BIOSKIN BPS-01L" manufactured by Regina Fashion Supply Co., Ltd., which is one of artificial skins, was evaluated. As a result, calculated average roughness Ra was 8.0 μm. As a result, it can be confirmed that calculated average roughness Ra of "BIOSKIN BPS-01L" manufactured by Regina Fashion Supply Co., Ltd. falls within the range of the calculated average roughness of the skin of the person and is close to the skin of the person. Note that, calculated average roughness Ra of "BIOSKIN BPS-01L" manufactured by Regina Fashion Supply Co., Ltd. was measured by using a profile analysis laser microscope manufactured by KEYENCE CORPORATION, and was measured at three points to obtain an average value thereof.

In the present exemplary embodiment, commercially available artificial skins having characteristics close to the skin of the person and in which the compressive stress at the time of being pushed at a depth of 2 mm is between 0.01 MPa and 0.05 MPa inclusive and calculated average roughness Ra is from 5 μm to 10 μm is used for specifying the coefficient of static friction of the skin wearing member. In the present exemplary embodiment, for example, "BIOSKIN BPS-01L" manufactured by Regina Fashion Supply Co., Ltd. is used as such an artificial skin.

The care device according to the present exemplary embodiment includes at least the skin wearing member having the Asker C hardness of 30 or less and the coefficient of static friction with respect to the artificial skin sheet having the characteristics (A) and (B) of 3 or less, and thus, it is possible to use the care device while feeling pleasantness when the care device is brought into contact with or worn on the skin. By feeling pleasantness, it is possible to obtain an effect of being relaxed, relieved, or easily sleeping.

The care device includes the skin wearing member having at least the Asker C hardness of 30 or less and the coefficient of static friction of 3 or less with respect to the artificial skin sheet having the characteristics of (A) and (B). The skin wearing member has a skin contact surface.

Fig. 1 is a cross-sectional view illustrating an example of the care device according to the first exemplary embodiment. Care device 100 illustrated in Fig. 1 has a configuration in which skin wearing member 1 is held by frame 3. As illustrated in Fig. 1, skin wearing member 1 may have, for example, a sheet shape. In this case, one surface of skin wearing member 1 is a portion coming into contact with the skin. Hereinafter, the portion of skin wearing member 1 coming into contact with the skin is referred to as skin contact surface 2. As illustrated in Fig. 1, skin wearing member 1 may be held by frame 3, or may have a structure in which at least some of supports 4 are coupled to frame 3. Support 4 may be, for example, an end portion of skin wearing member 1.

Since a shape of skin wearing member 1 of care device 100 according to the present exemplary embodiment is appropriately determined in accordance with a portion of the human body with or on which the care device is brought into contact or worn, the shape of the skin wearing member is not particularly limited. As in skin wearing member 1 illustrated in Fig. 1, in care device 100 according to the present exemplary embodiment, skin wearing member 1 may have, for example, a sheet shape.

A tensile strength of skin wearing member 1 may be more than, for example, 1 N/mm$^2$. The tensile strength of skin wearing member 1 is more than 1 N/mm$^2$, and thus, the skin wearing member can be stably used without being damaged even though the skin wearing member is repeatedly used even in a case where the skin wearing member is worn on the skin under tension.

Skin wearing member 1 may include at least an elastomer, and may be made of, for example, a polymer material containing an elastomer. Skin wearing member 1 may be made of only an elastomer. For example, skin wearing member 1 may be a single-layer sheet-shaped object made of an elastomer.

In the present exemplary embodiment, skin wearing member 1 may have a multilayer structure including a plurality of layers. Fig. 2 is a cross-sectional view illustrating an example of a skin wearing member having a multilayer structure. As illustrated in Fig. 2, skin wearing member 1 may include, for example, base member 11 containing an elastomer, and film 12 having a thickness of 100 μm or less disposed in at least region 11a of a surface of base member 11 facing the skin. Skin wearing member 1 may further include film 13 disposed in region 11b not facing the skin on the surface of base member 11. Film 13 may have a thickness of 100 μm or less. In a case where base member 11 is, for example, a sheet-shaped material, region 11a facing the skin on the surface of base member 11 corresponds to one principal surface (hereinafter, may be referred to as a "first principal surface") of base member 11, and region 11b not facing the skin on the surface of base member 11 corresponds to the other principal surface of base member 11, that is, a second principal surface facing the first principal surface. Here, the principal surface is a surface having a largest area. The second principal surface is a surface opposite to the first principal surface.

Skin wearing member 1 includes film 12 disposed in at least region 11a of the surface of base member 11 facing the skin as described above, and thus, it is easy to reduce the coefficient of static friction of the surface of skin wearing member 1 coming into contact with the skin. In addition, the strength of skin wearing member 1 can be improved by providing film 12. As a result, skin wearing member 1 is less likely to be broken, and the long-term durability of skin wearing member 1 is improved, such as being strong against friction. Film 13 is further provided, and thus, the strength of skin wearing member 1 can be further improved. As a result, the long-term durability of skin wearing member 1 can be further improved.

A thickness of film 12 is less than or equal to 100 μm, and thus, it is possible to obtain pleasantness in a case where skin wearing member 1 comes into contact with the skin. When the thickness of film 12 is less than or equal to 50 μm, higher pleasantness can be felt. When the thickness is less than or equal to 30 μm, higher pleasantness can be obtained with a natural touch feeling. A thickness of film 13 is not particularly limited, but for example, it is desirable that the thickness is not too thick such that skin wearing member 1 has flexibility to follow a surface shape of the skin when the care device is worn. The thickness of film 13 may be, for example, less than or equal to 100 μm.

The adhesion of film 12 to a surface of base member 11 may be performed by using an adhesive force of the elastomer contained in base member 11, an intermolecular force between the elastomer and film 12, or a van der Waals force between the elastomer and film 12. Alternatively, base member 11 and film 12 may be welded by base heat welding, or an adhesive layer may be provided on film 12, and film 12 may adhere to base member 11 with the adhesive layer. A pressure-sensitive adhesive, a moisture curable adhesive such as a cyanoacrylate adhesive or a silicone rubber adhesive, a solvent volatilization adhesive, a thermosetting adhesive, a two-liquid mixing adhesive, a hot melt adhesive, or the like can be used as the adhesive layer. The adhesion of film 13 to the surface of base member 11 is also described in the same manner as film 12.

Examples of the materials of film 12 and film 13 include thermoplastic elastomers such as polystyrene, polyurethane, polyester, polyolefin, acrylic resin, and polyvinyl chloride.

An adhesive force between base member 11 and film 12 is desirably more than or equal to 0.3 N in a peel test at a width of 15 mm. In the present exemplary embodiment, the peel test of base member 11 and film 12 is performed according to a test method of ASTM F88. The adhesive force between base member 11 and film 12 is more than or equal to 0.3 N, and thus, it is possible to obtain an effect of suppressing occurrence of appearance defects such as air bubbles at an interface due to peeling of film 12 from base member 11 during use.

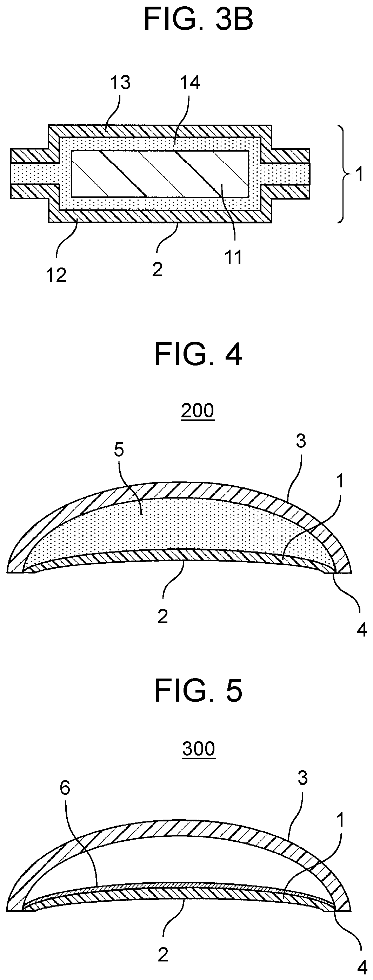

The entire surface of base member 11 may be covered with a film. That is, base member 11 may be sealed with a film. Figs. 3A and 3B are cross-sectional views illustrating a configuration in which base member 11 is sealed with a film in skin wearing member 1. Such a sealing structure can be obtained, for example, by bonding film 12 and film 13 to each other at a side surface portion of base member 11. Since care device 100 that is brought into contact with or worn on the skin is used while coming into contact with the skin, it is required to have high water resistance and oil resistance to moisture such as sweat on the skin, oil components such as sebum, and cosmetic components to be applied to the skin. Entire base member 11 containing the elastomer is sealed with the film, and thus, the film is hardly peeled off from the end portion of skin wearing member 1 even in a case where the base member is immersed in water, oil, or the like, or even in a case where a strong force is applied. As illustrated in Fig. 3A, film 12 and film 13 may be directly bonded to each other. Such bonding may be, for example, thermal welding. In addition, as illustrated in Fig. 3B, base member 11, film 12, and film 13 may be bonded to each other by adhesive layer 14, and film 12 and film 13 may be bonded to each other with adhesive layer 14 interposed therebetween at the side surface portion of base member 11. Examples of the adhesive layer include a pressure-sensitive adhesive, a moisture curable adhesive such as a cyanoacrylate adhesive or a silicone rubber adhesive, a solvent volatilization adhesive, a thermosetting adhesive, a two-liquid mixing adhesive, and a hot melt adhesive.

In the present exemplary embodiment, the skin wearing member may include, for example, the base member containing the elastomer, and a coating containing powder disposed in at least the region of the surface of the base member facing the skin. The coating containing the powder is performed, and thus, it is possible to feel a more smooth touch and to obtain high pleasantness. Examples of a material of the powder include inorganic powders such as zinc oxide, titanium oxide, talc, kaolin, mica, silica, titanium oxide, and iron oxide, and organic powders such as silicone resins, polyethylene, polypropylene, polyolefin, and acrylic. The coating containing the powder may be formed by directly attaching the powder to the base member, or may be formed by applying a coating material obtained by mixing the powder with a resin to the base member.

A pushing depth in a perpendicular direction when a load of 1 N is applied to the surface of skin contact surface 2 of skin wearing member 1 in the perpendicular direction by using a pushing jig having a cylindrical shape and having a diameter of 5 mm is desirably more than or equal to 1 mm. Note that, the pushing depth is measured for skin wearing member 1 in a state of being installed in care device 100. For example, in a case where skin wearing member 1 is installed in a state of being held by frame 3 as illustrated in Fig. 1, the pushing depth is a value measured for skin wearing member 1 in a state of being held by frame 3. When care device 100 is brought into contact with or worn on the skin, a load is applied to skin wearing member 1, and skin contact surface 2 is easily deformed in accordance with the shape of the skin to fit the skin. Skin contact surface 2 fits the skin, and thus, the person strongly feels the touch feeling of skin contact surface 2 of skin wearing member 1. The pushing depth of skin wearing member 1 measured under the above conditions is more than or equal to 1 mm, and thus, skin wearing member 1 is easily fitted to the skin, and high pleasantness can be felt. When the pushing depth in the perpendicular direction when the load of 1 N is applied to the surface of skin contact surface 2 of skin wearing member 1 in the perpendicular direction by using the cylindrical pushing jig having a diameter of 5 mm is more than or equal to 5 mm, skin contact surface 2 fits the skin more, and higher pleasantness can be felt.

The material that can be used as the elastomer contained in skin wearing member 1 is, for example, silicone, polystyrene, polyurethane, polyester, polyolefin, acrylic, polyvinyl chloride, or the like. The elastomer contained in skin wearing member 1 may be, for example, a gel containing polystyrene as a main material, a gel containing silicone as a main material, or silicone rubber. The gel containing polystyrene as the main material means a gel in which the most contained component is polystyrene. The same applies to the gel containing silicone as the main material. The elastomer is the gel containing polystyrene as the main material, the gel containing silicone as the main material, or silicone rubber, and thus, it is possible to obtain a soft and pleasant touch feeling while having high safety to a living body and being relatively inexpensive.

In the first exemplary embodiment, skin wearing member 1 may be made of a polymer material containing an elastomer. For example, the polymer material may include, as a component other than the elastomer, a polyolefin resin, a vinyl-based resin, an acryl-based resin, a polyester-based resin, a polycarbonate-based resin, a polystyrene-based resin, a polyamide resin, an epoxy-based resin, or the like.

Care device 100 according to the present exemplary embodiment may be, for example, a care device that is brought into contact with or worn on the face or the skin around the neck. Care device 100 includes skin wearing member 1 described above, and thus, pleasant stimulation can be obtained by bringing care device 100 into contact with the face or the skin around the neck or wearing the care device. A cosmetic effect such as beauty treatment can be obtained for the face or the skin around the neck.

Care device 100 according to the present exemplary embodiment may be, for example, a care device that is brought into contact with or worn on the skin around the eye. Care device 100 includes skin wearing member 1 described above, and thus, pleasant stimulation can be obtained by bringing care device 100 into contact with the skin around the eye or wearing the care device and eye fatigue can be removed. A cosmetic effect such as beauty treatment can be obtained for the skin around the eye. In addition, stimulation applied on the skin around the eye provides a relaxation feeling and an effect of promoting sleep.

A mechanism designed to give an appropriate physical stimulation or the like is provided in care device 100 in accordance with the portion of the human body to be brought into contact with or worn on. Note that, such a mechanism can use a mechanism used in a known care device for each portion of the human body, such as a care device for the face, a care device for the skin around the neck, and a care device for the skin around the eye.

[1-2. Operation]

Care device 100 having the above-described configuration can be brought into contact with or worn on the skin of the person via skin wearing member 1, and can give appropriate physical stimulation or the like corresponding to the portion of the human body to be brought into contact with or worn on. When care device 100 is used, the person comes into contact with skin wearing member 1. Accordingly, care device 100 can obtain an appropriate beauty treatment effect and massage effect with improvement of the pleasantness when the care device is brought into contact with the skin and when the care device is worn on the skin.

[1-3. Effects and the like]

As described above, care device 100 according to the first exemplary embodiment is the care device that is brought into contact with or worn on the skin. Care device 100 according to the first exemplary embodiment includes skin wearing member 1 containing the elastomer. Skin wearing member 1 has the Asker C hardness of 30 or less and the coefficient of static friction of 3 or less. In the first exemplary embodiment, the Asker C hardness and the coefficient of static friction of skin wearing member 1 are the Asker C hardness and the coefficient of static friction of the surface of skin wearing member 1 coming into contact with the skin when care device 100 is in contact with or worn on the skin. Here, the coefficient of static friction of skin wearing member 1 is the coefficient of static friction with respect to the artificial skin sheet having the characteristics (A) and (B). For example, "BIOSKIN BPS-01L" manufactured by Regina Fashion Supply Co., Ltd. can be used as such an artificial skin sheet.

According to such a configuration, care device 100 according to the first exemplary embodiment can improve the pleasantness when the care device is brought into contact with the skin and when the care device is worn on the skin.

In the first exemplary embodiment, the tensile strength of skin wearing member 1 may be more than, for example, $1 \text{ N/mm}^2$. The tensile strength of skin wearing member 1 is more than $1 \text{ N/mm}^2$, and thus, the skin wearing member can be stably used without being damaged even though the skin wearing member is repeatedly used even in a case where the skin wearing member is worn on the skin under tension.

In the first exemplary embodiment, skin wearing member 1 may include, for example, base member 11 containing the elastomer, and film 12 having the thickness of 100 $\mu$m or less disposed in at least region 11a of the surface of base member 11 facing the skin. Skin wearing member 1 includes film 12 disposed in at least region 11a of the surface of base member 11 facing the skin, and thus, it is easy to reduce the coefficient of static friction of the surface of skin wearing member 1 coming into contact with the skin. In addition, film 12 is provided, and thus, the strength of skin wearing member 1 can be improved. As a result, skin wearing member 1 is less likely to be broken, and the long-term durability of skin wearing member 1 is improved, such as being strong against friction.

In the first exemplary embodiment, the adhesive force between base member 11 and film 12 is desirably more than or equal to 0.3 N in the peel test at the width of 15 mm. The adhesive force between base member 11 and film 12 is more than or equal to 0.3 N, and thus, it is possible to obtain an effect of suppressing occurrence of appearance defects such as air bubbles at an interface due to peeling of film 12 from base member 11 during use.

In the first exemplary embodiment, the entire surface of base member 11 may be covered with the film. That is, base member 11 may be sealed with a film. Entire base member 11 containing the elastomer is sealed with the film, and thus, the film is hardly peeled off from the end portion of skin wearing member 1 even in a case where the base member is immersed in water, oil, or the like, or even in a case where a strong force is applied.

In the first exemplary embodiment, care device 100 may further include the coating containing powder disposed on the surface of skin contact surface 2 of skin wearing member 1. The coating containing the powder is performed, and thus, it is possible to feel a more smooth touch and to obtain high pleasantness.

In the first exemplary embodiment, the pushing depth in the perpendicular direction when the load of 1 N is applied to the surface of skin contact surface 2 of skin wearing member 1 in the perpendicular direction by using the cylindrical pushing jig having the diameter of 5 mm is desirably more than or equal to 1 mm. According to this configuration, when care device 100 is brought into contact with or worn on the skin, the load is applied to skin wearing member 1, and skin contact surface 2 is easily deformed according to the shape of the skin to fit the skin. Skin contact surface 2 fits the skin, and thus, the person strongly feels the touch feeling of skin contact surface 2 of skin wearing member 1. The pushing depth of skin wearing member 1 measured under the above conditions is more than or equal to 1 mm, and thus, skin wearing member 1 is easily fitted to the skin, and high pleasantness can be felt. When the pushing depth in the perpendicular direction when the load of 1 N is applied to the surface of skin contact surface 2 of skin wearing member 1 in the perpendicular direction by using the cylindrical pushing jig having the diameter of 5 mm is more than or equal to 5 mm, skin contact surface 2 fits the skin more, and higher pleasantness can be felt.

In the first exemplary embodiment, the elastomer contained in skin wearing member 1 may be, for example, the gel using polystyrene as the main material, the gel using silicone as the main material, or the silicone rubber. The elastomer is the gel containing polystyrene as the main material, the gel containing silicone as the main material, or silicone rubber, and thus, it is possible to obtain a soft and pleasant touch feeling with high safety for a living body at relatively low cost.

In the first exemplary embodiment, skin wearing member 1 may be made of a polymer material containing an elastomer. With this configuration, it is possible to obtain a soft and pleasant touch feeling at relatively low cost.

In the first exemplary embodiment, care device 100 may be, for example, the care device that is brought into contact with or worn on the face or the skin around the neck. Care device 100 includes skin wearing member 1 described above, and thus, pleasant stimulation can be obtained by bringing care device 100 into contact with the face or the skin around the neck or wearing the care device. A cosmetic effect such as beauty treatment can be obtained for the face or the skin around the neck.

In the first exemplary embodiment, care device 100 may be, for example, the care device that is brought into contact with or worn on the skin around the eye. Care device 100 includes skin wearing member 1 described above, and thus, pleasant stimulation can be obtained by bringing care device 100 into contact with the skin around the eye or wearing the care device and eye fatigue can be removed. A cosmetic effect such as beauty treatment can be obtained for the skin around the eye.

(Other exemplary embodiments)

As described above, the exemplary embodiment has been described as an example of the technology disclosed in the present application. However, the technology of the present disclosure is not limited thereto, but may be applicable to exemplary embodiments with changes, additions, omissions, or the like. In addition, the components described in the above exemplary embodiment may be combined to make an additional exemplary embodiment.

Therefore, another exemplary embodiment will be illustrated below.

Fig. 4 is a cross-sectional view illustrating a first modification of the care device according to the first exemplary embodiment. As illustrated in Fig. 4, care device 200 as the first modification has a configuration in which underlying member 5 is further provided with respect to care device 100 illustrated in Fig. 1. In care device 200, in a state where skin wearing member 1 is worn on the skin, underlying member 5 is provided at a position opposite to the skin with respect to skin wearing member 1 and comes into contact with at least a part of a portion of skin wearing member 1 not coming into contact with the skin. The portion of skin wearing member 1 not coming into contact with the skin is, for example, a surface of skin wearing member 1 opposite to skin contact surface 2. A 10% compressive stress of underlying member 5 is less than or equal to 0.2 MPa. The 10% compressive load of underlying member 5 is less than or equal to 0.2 MPa, and thus, underlying member 5 is easily compressed when a load is applied to a surface of skin contact surface 2 of skin wearing member 1. Accordingly, the surface of skin contact surface 2 of skin wearing member 1 is easily deformed, and skin contact surface 2 can be deformed according to the shape of the skin when care device 200 is brought into contact with or worn on the skin. As a result, skin contact surface 2 more easily fits the skin, and the person strongly feels the touch feeling of skin contact surface 2 of skin wearing member 1 as skin contact surface 2 fits the skin. As described above, underlying member 5 is provided, and thus, skin wearing member 1 easily fits the skin. As a result, high pleasantness can be felt. When the 10% compressive stress of underlying member 5 is less than or equal to 0.013 MPa, underlying member 5 is more easily compressed when a load is applied to the surface of skin contact surface 2 of skin wearing member 1. As a result, skin wearing member 1 fits the skin more, and higher pleasantness can be felt. Note that, the 10% compressive stress of underlying member 5 can be measured by methods described in examples to be described later.

An area of skin contact surface 2 of skin wearing member 1 is desirably more than 4 cm$^2$. When a contact area with the skin is large, the touch feeling of skin wearing member 1 can be strongly felt. The area of skin contact surface 2 of skin wearing member 1 is more than 4 cm$^2$, and thus, in a case where care device 100 comes into contact with or worn on the skin, it is easy to feel the touch feeling of skin wearing member 1. As a result, high pleasantness can be felt. When the area of skin contact surface 2 of skin wearing member 1 is more than or equal to 9 cm$^2$, the touch feeling of skin wearing member 1 is more easily felt, and higher pleasantness can be felt. When the area of skin contact surface 2 of skin wearing member 1 is more than or equal to 15 cm$^2$, the touch feeling of skin wearing member 1 is more easily felt, and higher pleasantness can be felt. When the area of skin contact surface 2 of skin wearing member 1 is more than or equal to 25 cm$^2$, the touch feeling of skin wearing member 1 is more easily felt, and higher pleasantness can be felt.

Fig. 5 is a cross-sectional view illustrating a second modification of the care device according to the first exemplary embodiment. As illustrated in Fig. 5, care device 300 as the second modification has a configuration in which heating element 6 is further provided with respect to care device 100 illustrated in Fig. 1. Care device 300 includes heating element 6 capable of continuously controlling a temperature. Heating element 6 capable of continuously controlling the temperature is provided, and thus, it is possible to use care device 300 while feeling a thermal sensation, and an effect of further improving pleasantness can be obtained. In addition, blood circulation is improved by warming the skin, and effects such as a cosmetic effect, a massage effect, and an improvement in metabolism can be obtained, or a relaxation effect can be obtained by feeling a thermal sensation. For example, an installation position of heating element 6 is not particularly limited, and the heating element may be installed at any part of care device 300. Further, as illustrated in Fig. 5, for example, heating element 6 may be provided at a position opposite to the skin with respect to skin wearing member 1 in a state where skin wearing member 1 is worn on the skin, and may be provided to come into contact with at least a part of the portion of skin wearing member 1 not coming into contact with the skin. The portion of skin wearing member 1 not coming into contact with the skin is, for example, the position opposite to the skin with respect to skin wearing member 1 in a state where skin wearing member 1 is worn on the skin, and is, for example, a surface of skin wearing member 1 opposite to skin contact surface 2. Heating element 6 is provided at a part of the portion of skin wearing member 1 not coming into contact with the skin, and thus, heating element 6 can directly heat skin wearing member 1. Accordingly, the thermal sensation can be efficiently given with low power. For example, heating element 6 may be installed at any part of care device 300, and heating element 6 may be provided to come into contact with a part of the portion not coming into contact with the skin of skin wearing member 1 only in a state where skin wearing member 1 is worn on the skin. In this case, heating element 6 is not installed on skin wearing member 1 in a state where the heating element is not worn on the skin, and thus, the touch feeling of skin wearing member 1 is improved at the time of being worn. As a result, the thermal sensation can be efficiently provided with low power after being worn. For example, an electric heating wire heater, an infrared heater, a far infrared heater, a hot water heater, a sheath heater, a Peltier element, or the like is used as the heating element.

A cooling controller that cools skin wearing member 1 may be further included with respect to care device 100 illustrated in Fig. 1. With this configuration, it is possible to impart a cooling sensation to the skin to increase pleasantness, and it is possible to impart a tightening cosmetic effect to the skin by a cooling sensation stimulation. For example, a Peltier element, water cooling, air cooling, a cold reserving pack, or the like can be used as the cooling controller.

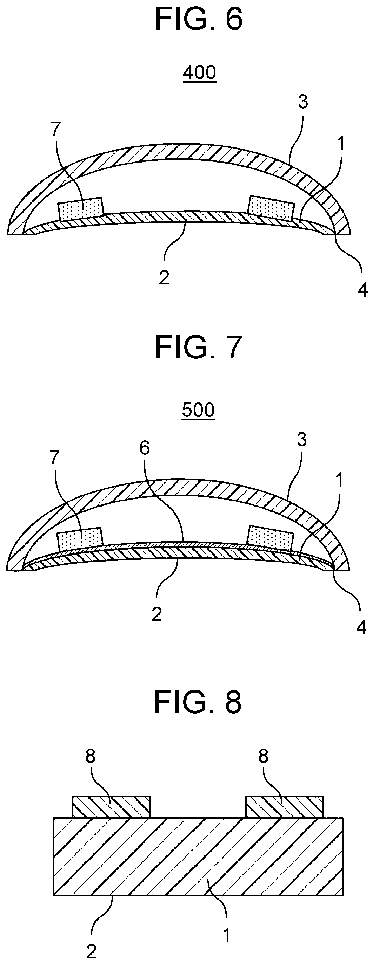

Fig. 6 is a cross-sectional view illustrating a third modification of the care device according to the first exemplary embodiment. As illustrated in Fig. 6, care device 400 as the third modification has a configuration in which vibrator 7 is further provided with respect to care device 100 illustrated in Fig. 1. Care device 400 includes vibrator 7 that vibrates skin wearing member 1. Vibrator 7 capable of vibrating skin wearing member 1 is provided, and thus, it is possible to use care device 400 while performing massage by vibration. As a result, it is possible to loosen muscles and the like in addition to an effect of improving the pleasantness. Thus, according to care device 400, an effect of reducing fatigue and a cosmetic effect can be obtained. For example, vibrator 7 may be installed at any part of care device 400. As illustrated in Fig. 6, for example, in a state where skin wearing member 1 is worn on the skin, vibrator 7 may be provided at a position opposite to the skin with respect to skin wearing member 1, and may be provided to come into contact with at least a part of the portion of skin wearing member 1 not coming into contact with the skin. The portion of skin wearing member 1 not coming into contact with the skin is, for example, the position opposite to the skin with respect to skin wearing member 1 in a state where skin wearing member 1 is worn on the skin, and is, for example, a surface of skin wearing member 1 opposite to skin contact surface 2. Vibrator 7 is provided at a part of the portion of skin wearing member 1 not coming into contact with the skin, and thus, vibration can be directly applied to skin wearing member 1. Accordingly, vibration can be efficiently applied with low power. For example, vibrator 7 may be installed at any part of the care device. Vibrator 7 may be provided such that vibrator 7 comes into contact with a part of the portion not coming into contact with the skin of skin wearing member 1 only in a state where skin wearing member 1 is worn on the skin. In this case, vibrator 7 is not installed on skin wearing member 1 in a state where the vibrator is not worn on the skin, and thus, the touch feeling of skin wearing member 1 is improved at the time of being worn. As a result, vibration can be efficiently applied with low power after being worn.

Fig. 7 is a cross-sectional view illustrating a fourth modification of the care device according to the first exemplary embodiment. As illustrated in Fig. 7, care device 500 according to a fourth modification has a configuration in which heating element 6 and vibrator 7 are further provided with respect to care device 100 illustrated in Fig. 1. Note that, heating element 6 and vibrator 7 are as described above. Care device 500 includes both heating element 6 and vibrator 7, and thus, care device 500 can be used while performing massage by vibration in addition to the thermal sensation. Accordingly, according to care device 500, the pleasantness is further improved, and the effect of reducing the fatigue and the cosmetic effect can be simultaneously obtained.

Fig. 8 is a cross-sectional view illustrating a fifth modification of the care device according to the first exemplary embodiment. As illustrated in Fig. 8, the care device as the fifth modification further includes vibration assist jig 8 with respect to care device 100 illustrated in Fig. 1. Vibration assist jig 8 is provided at a portion of skin wearing member 1 not coming into contact with the skin in a state where skin wearing member 1 is worn on the skin. The portion of skin wearing member 1 not coming into contact with the skin is, for example, the position opposite to the skin with respect to skin wearing member 1 in a state where skin wearing member 1 is worn on the skin, and is, for example, a surface of skin wearing member 1 opposite to skin contact surface 2. Vibration assist jig 8 is made of, for example, a solid material having a mass per unit area of $0.02 \text{ g/cm}^2$ or more and has a mass of 500 g or less. When such vibration assist jig 8 is installed, the vibration is transmitted to vibration assist jig 8 to vibrate vibration assist jig 8, and when the mass per unit area is large, the vibration applied to the skin at a portion where vibration assist jig 8 is installed becomes strong. When vibration assist jig 8 made of the solid material having the mass per unit area of $0.02 \text{ g/cm}^2$ or more is installed, the vibration applied to the skin at the portion where vibration assist jig 8 is installed becomes strong, and thus, a vibration feeling when the care device is brought into contact with or worn on the skin becomes strong. In addition, when the entire mass of vibration assist jig 8 increases, the mass of the care device itself increases. The person feels a weight when the care device is worn, and feels a sense of discomfort. In a case where the entire mass of vibration assist jig 8 is less than or equal to 500 g, the mass of the care device is not large, and thus, the care device can be comfortably worn. Vibration assist jig 8 may be a solid material having a mass per unit area of $0.03 \text{ g/cm}^2$ or more. In this case, the vibration applied to the skin of the portion where vibration assist jig 8 is installed becomes strong. Vibration assist jig 8 may be a solid material having a mass per unit area of $0.2 \text{ g/cm}^2$ or more. In this case, the vibration applied to the skin of the portion where vibration assist jig 8 is installed becomes stronger. As a result, the vibration feeling of the portion where vibration assist jig 8 is installed when the care device is brought into contact with or worn on the skin becomes strong, and it is possible to strongly stimulate a part of the skin. In addition, the entire mass of vibration assist jig 8 may be less than or equal to 100 g. In a case where the entire mass of vibration assist jig 8 is less than or equal to 100 g, the mass of the care device is reduced, and the care device can be more comfortably worn.

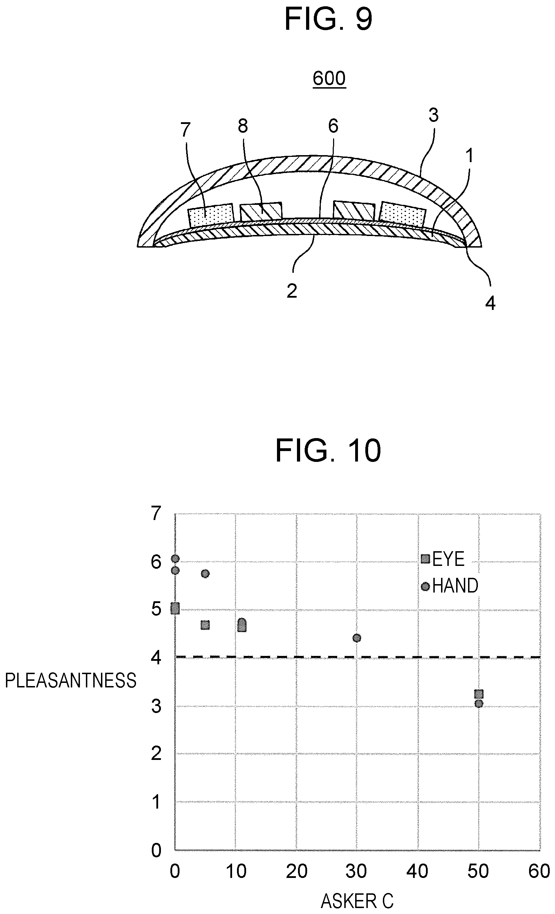

Fig. 9 is a cross-sectional view illustrating a six modification of the care device according to the first exemplary embodiment. As illustrated in Fig. 9, care device 600 according to the sixth modification further includes vibrator 7 and vibration assist jig 8 with respect to care device 100 illustrated in Fig. 1. Note that, vibrator 7 and vibration assist jig 8 are as described above. Care device 600 includes both vibrator 7 and vibration assist jig 8, and thus, it is possible to enhance the vibration applied to the skin of the portion where vibration assist jig 8 is installed with respect to the vibration by vibrator 7. Accordingly, care device 600 can apply strong stimulation to a part of the skin by partially strengthening the vibration by vibrator 7 by vibration assist jig 8.

A pressure change unit that changes the pressure applied to skin wearing member 1 may be further included with respect to care device 100 illustrated in Fig. 1. With this configuration, the pressure can be applied to the skin, the pleasantness can be further improved, and the massage effect can be given. For example, an airbag, a jig operated by a motor, or the like can be used as the pressure change unit.

A tensile force change unit that changes a tensile force applied to skin wearing member 1 may be further included with respect to care device 100 illustrated in Fig. 1. With this configuration, an optimum tensile force is applied to the skin wearing member to increase a fitting property to the skin or to suppress an excessive tensile force. In this way, it is possible to prevent the skin wearing member from being broken. An actuator, a frame or a belt capable of adjusting a length, or the like that can reversibly apply the tensile force to the skin wearing member by being installed at an end of the skin wearing member and changing the length can be used as the tensile force change unit.

A skin portion detection unit that detects the portion of the skin with or on which care device 100 is brought into contact or worn, and a temperature controller that controls a temperature by heating or cooling skin wearing member 1 in accordance with a result detected by the skin portion detection unit may be further included with respect to care device 100 illustrated in Fig. 1. With this configuration, it is possible to give optimum thermal and cold stimulations to the portion of the skin, to further improve the pleasantness, and to promote a blood flow by repeating heating and cooling, and to give a cosmetic effect. For example, light such as a camera and infrared rays can be used as the skin portion detection unit. As an example, the temperature controller holds the heating element and elements (for example, a Peltier element, water cooling, air cooling, or a cold reserving pack) used for the cooling controller illustrated above. In this way, the temperature controller can retain both heating and cooling functions.

Care device 400 including vibrator 7 illustrated in Fig. 6 may further include a skin portion detection unit that detects the portion of the skin with or on which care device 400 is brought into contact or worn, and a system that controls a vibration amount of vibrator 7 in accordance with a result detected by the skin portion detection unit. With this configuration, an optimum vibration can be given to the skin portion, the pleasantness can be further improved, and the massage effect can be given. The units illustrated above can be used as the skin portion detection unit. The system that controls the vibration amount includes, for example, a device capable of controlling the vibration amount or the vibration pattern of the vibrator.

A skin portion detection unit that detects the portion of the skin with or on which care device 100 is brought into contact or worn, and a pressure change unit that changes the pressure applied to skin wearing member 1 in accordance with a result detected by the skin portion detection unit in accordance with a result detected by the skin portion detection unit may be further included with respect to care device 100 illustrated in Fig. 1. With this configuration, the optimum pressure can be applied to the portion of the skin, and the pleasantness and the massage effect are further enhanced. The units illustrated above can be used as the skin portion detection unit and the pressure change unit.

A skin portion detection unit that detects the portion of the skin with or on which care device 100 is brought into contact or worn, and a tensile force change unit that changes a tensile force applied to skin wearing member 1 in accordance with a result detected by the skin portion detection unit may be further included with respect to care device 100 illustrated in Fig. 1. With this configuration, it is possible to apply an optimum tensile force to the skin portion, and it is possible to prevent the skin wearing member from being broken by enhancing a fitting property to the skin or suppressing an excessive tensile force even though the shape of the skin, a position and a size of a wearing unit, or the shape of the skin changes. The units illustrated above can be used as the skin portion detection unit and the tensile force change unit.

(Appendix)

The above description of the exemplary embodiment discloses the following technologies.

(Technology 1)

A care device that is brought into contact with or worn on a skin, including a skin wearing member containing an elastomer,
in which the skin wearing member has an Asker C hardness of 30 degrees or less and a coefficient of static friction of 3 or less.

Here, the coefficient of static friction is a coefficient of static friction for an artificial skin sheet that satisfies the following characteristics (A) and (B).

(A) A compressive stress of the artificial skin sheet at a time of being pushed in a thickness direction at a depth of 2 mm is between 0.01 MPa and 0.05 MPa inclusive.
(B) Calculated average roughness Ra of a surface of the artificial skin sheet is from 5 $\mu$m to 10 $\mu$m.

With this configuration, the care device according to Technology 1 can improve pleasantness when the care device is brought into contact with the skin and when the care device is worn on the skin.

(Technology 2)

The care device according to Technology 1, in which a tensile strength of the skin wearing member is more than 1 N/mm$^2$.
With this configuration, the care device according to Technology 2 can be stably used even in a case where the care device is repeatedly used without being damaged even though the care device is worn on the skin with tension.

(Technology 3)

The care device according to Technology 1 or 2, in which

the skin wearing member includes
a base member containing the elastomer, and
a film having a thickness of 100 $\mu$m or less, the film being disposed in at least a region of a surface of the base member facing the skin.

With this configuration, the care device according to Technology 3 can easily reduce the coefficient of static friction of the surface of the skin wearing member coming into contact with the skin. In addition, the film is provided, and thus, the strength of the skin wearing member can be improved. As a result, the skin wearing member is less likely to be broken, and the long-term durability of the skin wearing member is improved, such as being strong against friction.

(Technology 4)

The care device according to Technology 3, in which an adhesive force between the base member and the film is more than or equal to 0.3 N in a peel test at a width of 15 mm.
With this configuration, it is possible to obtain an effect of suppressing occurrence of appearance defects such as air bubbles at an interface due to peeling of the film from the base member during use.

(Technology 5)

The care device according to Technology 3 or 4, in which the film covers an entire surface of the base member.
With this configuration, the film is hardly peeled off from the end portion of the skin wearing member even in a case where the base member is immersed in water, oil, or the like, or even in a case where a strong force is applied.

(Technology 6)

The care device according to any one of Technologies 1 to 5, in which

the skin wearing member includes
a base member containing the elastomer, and
a coating containing powder disposed in at least a region of a surface of the base member facing the skin.

With this configuration, it is possible to feel a more smooth touch feeling, and to obtain high pleasantness.

(Technology 7)

The care device according to any one of Technologies 1 to 6, in which
a pushing depth in a direction perpendicular to a surface of a portion of the skin wearing member coming into contact with the skin when a load of 1 N is applied to the surface in the perpendicular direction by using a pushing jig having a cylindrical shape and having a diameter of 5 mm is more than or equal to 1 mm.

With this configuration, the skin wearing member easily fits the skin, and high pleasantness can be felt.

(Technology 8)

The care device according to any one of Technologies 1 to 7, further including an underlying member,

in which the underlying member is provided at a position opposite to the skin with respect to the skin wearing member and comes into contact with at least a part of a portion of the skin wearing member not coming into contact with the skin in a state where the skin wearing member is worn on the skin, and
a 10% compressive stress of the underlying member is less than or equal to 0.2 MPa.

With this configuration, the skin wearing member easily fits the skin, and high pleasantness can be felt.

(Technology 9)

The care device according to any one of Technologies 1 to 8, in which an area of a skin wearing surface of the skin wearing member is more than 4 cm$^2$.

With this configuration, it is easy to feel the touch feeling of the skin wearing member, and high pleasantness can be felt.

(Technology 10)

The care device according to any one of Technologies 1 to 9, in which the elastomer is a gel containing polystyrene as a main material, a gel containing silicone as a main material, or silicone rubber.

With this configuration, it is possible to provide a care device capable of obtaining a soft and pleasant touch feeling with high safety for a living body at relatively low cost.

(Technology 11)

The care device according to any one of Technologies 1 to 10, in which the skin wearing member is made of a polymer material containing the elastomer.

With this configuration, it is possible to provide a care device capable of obtaining a soft and pleasant touch feeling at relatively low cost.

(Technology 12)

The care device according to any one of Technologies 1 to 11, further including a heating element configured to continuously control a temperature.

With this configuration, it is possible to use the care device while feeling a thermal sensation, and an effect of further improving pleasantness can be obtained. In addition, blood circulation is improved by warming the skin, and effects such as a cosmetic effect, a massage effect, and an improvement in metabolism can be obtained, or a relaxation effect can be obtained by feeling a thermal sensation.

(Technology 13)

The care device according to any one of Technologies 1 to 12, further including a cooling controller that cools the skin wearing member to control a temperature.

With this configuration, it is possible to impart a cooling sensation to the skin to increase pleasantness, and it is possible to impart a tightening cosmetic effect to the skin by a cooling sensation stimulation.

(Technology 14)

The care device according to any one of Technologies 1 to 13, further including a vibrator that vibrates the skin wearing member.

With this configuration, it is possible to use the care device while performing massage by vibration, and it is possible to loosen muscles and the like in addition to the effect of improving the pleasantness.

(Technology 15)

The care device according to any one of Technologies 1 to 14, further including a vibration assist jig,

in which the vibration assist jig is provided at a portion of the skin wearing member not coming into contact with the skin in a state where the skin wearing member is worn on the skin, and
the vibration assist jig is made of a solid material having a mass per unit area of 0.02 g/cm$^2$ or more and has a mass of 500 g or less.

With this configuration, the vibration is transmitted to the vibration assist jig to vibrate the vibration assist jig, and when the mass per unit area is large, the vibration applied to the skin at the portion where the vibration assist jig is installed becomes strong. When the vibration assist jig made of the solid material having the mass per unit area of 0.02 g/cm$^2$ or more is installed, the vibration applied to the skin at the portion where the vibration assist jig is installed becomes strong, and thus, the vibration feeling when the care device is brought into contact with or worn on the skin becomes strong.

(Technology 16)

The care device according to any one of Technologies 1 to 15, further including a pressure change unit that changes a pressure applied to the skin wearing member.
With this configuration, the pressure can be applied to the skin, the pleasantness can be further improved, and the massage effect can be given.

(Technology 17)

The care device according to any one of Technologies 1 to 16, in which a tensile force change unit that changes a tensile force applied to the skin wearing member.
With this configuration, an optimum tensile force is applied to the skin wearing member, and thus, it is possible to prevent the skin wearing member from being broken by increasing a fitting property to the skin or suppressing an excessive tensile force.

(Technology 18)

The care device according to any one of Technologies 1 to 17, further including

a skin portion detection unit that detects a portion of the skin with or on which the care device is brought into contact or worn, and
a temperature controller that heats or cools the skin wearing member to control a temperature in accordance with a result detected by the skin portion detection unit.

With this configuration, it is possible to give optimum thermal and cold stimulations to the portion of the skin, to further improve the pleasantness, and to promote a blood flow by repeating heating and cooling, and to give a cosmetic effect.

(Technology 19)

The care device according to Technology 14, further including

a skin portion detection unit that detects a portion of the skin with or on which the care device is brought into contact or worn, and
a system that changes a vibration amount of the vibrator in accordance with a result detected by the skin portion detection unit.

With this configuration, an optimum vibration can be given to the skin portion, the pleasantness can be further improved, and the massage effect can be given.

(Technology 20)

The care device according to Technology 16, further including a skin portion detection unit that detects a portion of the skin with or on which the care device is brought into contact or is worn,

in which the pressure change unit changes a pressure applied to the skin wearing member in accordance with a result detected by the skin portion detection unit.

With this configuration, the optimum pressure can be applied to the portion of the skin, and the pleasantness and the massage effect are further enhanced.

(Technology 21)

The care device according to Technology 17, further including a skin portion detection unit that detects a portion of the skin with or on which the care device is brought into contact or is worn,

in which the tensile force change unit changes a tensile force applied to the skin wearing member in accordance with a result detected by the skin portion detection unit.

With this configuration, it is possible to apply an optimum tensile force to the skin portion, and it is possible to prevent the skin wearing member from being broken by enhancing a fitting property to the skin or suppressing an excessive tensile force even though the shape of the skin, a position and a size of a wearing unit, or the shape of the skin changes.

(Technology 22)

The care device according to any one of Technologies 1 to 21, in which the care device is brought into contact with or worn on a face or a skin around a neck.

The pleasant stimulation can be obtained by bringing the care device into contact with the face or the skin around the neck or wearing the care device, and a cosmetic effect such as a beauty treatment can be obtained for the face or the skin around the neck.

(Technology 23)

The care device according to any one of Technologies 1 to 22, in which the care device is brought into contact with or worn on a skin around an eye.

The pleasant stimulation can be obtained by bringing the care device into contact with the skin around the eye or wearing the care device and eye fatigue can be removed. A cosmetic effect such as beauty treatment can be obtained for the skin around the eye. In addition, stimulation applied on the skin around the eye provides a relaxation feeling and an effect of promoting sleep.

Examples

The care device of the present disclosure will be described in more detail by way of examples. The care device of the present disclosure is not limited to the following examples.

<Hardness measurement>

For the evaluation of the Asker C hardness, measurement was performed at a room temperature by using "Asker rubber hardness meter type C" manufactured by Kobunshi Keiki Co., Ltd.

<Measurement of coefficient of static friction>

A sliding piece with constant vertical load W was placed on a surface of a sample, frictional force F acting when the sliding piece was moved in a horizontal direction at a constant speed was measured, and coefficient of static friction $\mu$ was calculated on the basis of the following Equation 1.

$$\mu = F/W \ \text{(Equation 1)}$$

In the measurement of the coefficient of static friction for the artificial skin, "BIOSKIN BPS-01L" manufactured by Regina Fashion Supply Co., Ltd. was used as an artificial skin for the sliding piece. The coefficient of static friction at a moving speed of 100 mm/min was measured by using force gauge systems ZTA-50N and MX2-500N-FA (manufactured by IMADA Co., Ltd.) under a condition in which a contact area of the sliding piece was 20 mm $\times$ 20 mm and vertical load W was 20 g.

<Example 1>

(Preparation of skin wearing member)

A silicone gel having a sheet shape (manufactured by TANAC Co., Ltd.) was prepared as a base member. A surface of the sheet was coated with powder containing talc as a main component (manufactured by WAKODO). As described above, the skin wearing member formed by applying the powder coating to the base member was prepared. In the skin wearing member, an Asker C hardness was 0 (C0), and a coefficient of static friction for the artificial skin was 1.5. This skin wearing member was used as a skin wearing member of Example 1.

(Evaluation)

A care device was produced by attaching the skin wearing member of Example 1 to an eye care device (eye care device manufactured by Panasonic Corporation) as a frame. In a state where the skin contact surface of the skin wearing member came into contact with the skin, the pleasantness of the touch feeling of the eye when the care device was worn on the eye was evaluated. Subjects were 16 men and women in their 20s to 50s, sat facing a reclining seat, and performed three sets of processes of wearing the care device on their eye, wearing the device for 10 seconds, and then removing the device. Thereafter, the subjects kept scores based on the following criteria, and average values of scores of the subjects were calculated.

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

Further, the pleasantness of the touch feeling when the skin contact surface of the skin wearing member in the care device was brought into contact with the hand was evaluated. The evaluation method was performed by the same method as the evaluation of the touch feeling of the eye by bringing the right hand of the subject into contact with the skin contact surface of the skin wearing member for 10 seconds.

In the care device of Example 1, the pleasantness of the touch feeling of the eye was 5.1 points, and the pleasantness of the touch feeling of the hand was 6.1 points.

<Example 2>

(Preparation of skin wearing member)

A styrene elastomer having a sheet shape (manufactured by Efu-Kei. Co., Ltd.) was prepared as a base member of the skin wearing member. A surface of the base member was coated with powder containing talc as a main component (manufactured by WAKODO). As described above, the skin wearing member formed by applying the powder coating to the base member was prepared. In the skin wearing member, an Asker C hardness was 0 (C0), and a coefficient of static friction for the artificial skin was 1.2. This skin wearing member was used as a skin wearing member of Example 2.

(Evaluation)

A care device was produced by the same method as in Example 1 except that the skin wearing member of Example 2 was used, and the pleasantness of the touch feeling of the eye and hand of the care device was evaluated. In the care device of Example 2, the pleasantness of the touch feeling of the eye was 5.0 points, and the pleasantness of the touch feeling of the hand was 5.8 points.

<Example 3>

(Preparation of skin wearing member)

A styrene elastomer having a sheet shape (manufactured by Kuraray Trading Co., Ltd.) was prepared as a base member. A film obtained by stacking a polyurethane-based polymer film having a thickness of 13 $\mu$m and a hot melt adhesive layer was installed on a surface of the base member such that the hot melt adhesive layer faced the elastomer. As described above, the skin wearing member in which the film was disposed on the surface of the base member was prepared. In the skin wearing member, an Asker C hardness was 11 (C11), and a coefficient of static friction for the artificial skin was 0.7. This skin wearing member was used as a skin wearing member of Example 3.

(Evaluation)

A care device was produced by the same method as in Example 1 except that the skin wearing member of Example 3 was used, and the pleasantness of the touch feeling of the eye and hand of the care device was evaluated. In the care device of Example 3, the pleasantness of the touch feeling of the eye was 4.6 points, and the pleasantness of the touch feeling of the hand was 4.8 points.

<Example 4>

(Preparation of skin wearing member)

A skin wearing member (manufactured by TANAC Co., Ltd.) in which an Asker C hardness was 5 (C5) and a coefficient of static friction for the artificial skin was 1.8 was prepared. The skin wearing member had a configuration in which a surface of a sheet was coated with a polyurethane resin on polyurethane having a sheet shape. In other words, polyurethane having a sheet shape (manufactured by TANAC Co., Ltd.) in which an Asker C hardness was 5 (C5) and a coefficient of static friction for the artificial skin was 1.8 was prepared as a skin wearing member of Example 4.

(Evaluation)

A care device was produced by the same method as in Example 1 except that the skin wearing member of Example 4 was used, and the pleasantness of the touch feeling of the eye and hand of the care device was evaluated. In the care device of Example 4, the pleasantness of the touch feeling of the eye was 4.7 points, and the pleasantness of the touch feeling of the hand was 5.8 points.

<Example 5>

(Preparation of skin wearing member)

A silicone rubber having a sheet shape (manufactured by TANAC Co., Ltd.) in which an Asker C hardness was 30 (C30) and a coefficient of static friction for the artificial skin was 2.1 was prepared as a skin wearing member of Example 5.

(Evaluation)

A care device was produced by the same method as in Example 1 except that the skin wearing member of Example 5 was used, and the pleasantness of the touch feeling of the hand of the care device was evaluated. In the care device of Example 5, the pleasantness of the touch feeling of the hand was 4.4 points.

<Comparative Example 1>

(Preparation of skin wearing member)

A silicone rubber having a sheet shape (manufactured by Kyowa Industrial co., LTD.) in which an Asker C hardness was 50 (C50) and a coefficient of static friction for the artificial skin was 1.3 was prepared as a skin wearing member of Comparative Example 1.

(Evaluation)

A care device was produced by the same method as in Example 1 except that the skin wearing member of Comparative Example 1 was used, and the pleasantness of the touch feeling of the eye and hand of the care device was evaluated. In the care device of Comparative Example 1, the pleasantness of the touch feeling of the eye was 3.3 points, and the pleasantness of the touch feeling of the hand was 3.1 points.

<Comparative Example 2>

(Preparation of skin wearing member)

A silicone gel having a sheet shape (manufactured by TANAC Co., Ltd.) in which an Asker C hardness was 0 (C0) and a coefficient of static friction for the artificial skin was 4.3 was prepared as a skin wearing member of Comparative Example 2.

(Evaluation)

A care device was produced by the same method as in Example 1 except that the skin wearing member of Comparative Example 2 was used, and the pleasantness of the touch feeling of the eye and hand of the care device was evaluated. In the care device of Comparative Example 2, the pleasantness of the touch feeling of the eye was 3.6 points, and the pleasantness of the touch feeling of the hand was 3.1 points.

<Comparative Example 3>

(Preparation of skin wearing member)

Polyurethane having a sheet shape (manufactured by TANAC Co., Ltd.) in which an Asker C hardness was 0 (C0) and a coefficient of static friction for the artificial skin was 6.5 was prepared as a skin wearing member of Comparative Example 3.

(Evaluation)

A care device was produced by the same method as in Example 1 except that the skin wearing member of Comparative Example 3 was used, and the pleasantness of the touch feeling of the eye and hand of the care device was evaluated. In the care device of Comparative Example 3, the pleasantness of the touch feeling of the eye was 2.9 points, and the pleasantness of the touch feeling of the hand was 1.8 points.

The results of Examples 1 to 5 are summarized in Table 1A, and the results of Comparative Examples 1 to 3 are summarized in Table 1B. In Examples 1 to 5, the score of the pleasantness of the touch feeling of the eye and hand of the care device was more than or equal to 4 points, and the pleasantness of the care device was high. On the other hand, in Comparative Examples 1 to 3, the score of the pleasantness of the touch feeling of the eye and hand of the care device was less than 4 points, and the pleasantness was low.

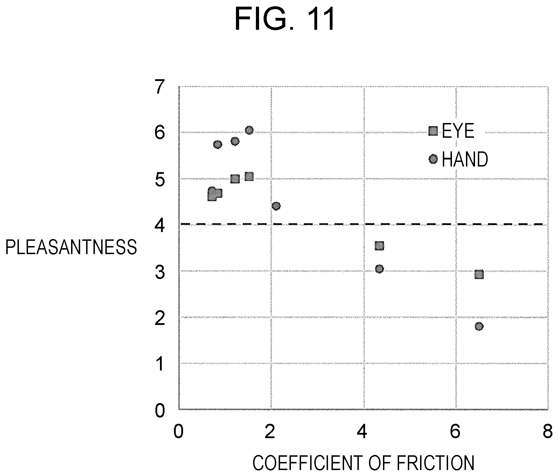

Fig. 10 is a graph representing a relationship between the Asker C hardness of the skin wearing member and the pleasantness of the touch feeling of the eye and hand when the care device is used. Fig. 11 is a graph representing a relationship between the coefficient of static friction of the skin wearing member for the artificial skin and the pleasantness of the touch feeling of the eye and hand when the care device is used.

Table 1A

| | | Example 1 | Example 2 | Example 3 | Example 4 | Example 5 |
|---|---|---|---|---|---|---|
| Elastomer | | Silicone Gel | Polystyrene | Polystyrene | Polyurethane | Silicone rubber |
| Characteristics of skin wearing member | Asker C hardness | 0 | 0 | 11 | 5 | 30 |
| | Coefficient of static friction for artificial skin | 1.5 | 1.2 | 0.7 | 1.8 | 2.1 |
| Evaluation of care device | Pleasantness of touch feeling of eye | 5.1 | 5.0 | 4.6 | 4.7 | - |
| | Pleasantness of touch feeling of hand | 6.1 | 5.8 | 4.8 | 5.8 | 4.4 |

Table 1B

| | | Comparative Example 1 | Comparative Example 2 | Comparative Example 3 |
|---|---|---|---|---|
| Elastomer | | Silicone rubber | Silicone Gel | Polyurethane |
| Characteristics of skin wearing member | Asker C hardness | 50 | 0 | 0 |
| | Coefficient of static friction for artificial skin | 1.3 | 4.3 | 6.5 |
| Evaluation of care device | Pleasantness of touch feeling of eye | 3.3 | 3.6 | 2.9 |
| | Pleasantness of touch feeling of hand | 3.1 | 3.1 | 1.8 |

<Example 6>

(Preparation of skin wearing member)

A film (manufactured by Kuraray Trading Co., Ltd.) obtained by stacking a polyurethane-based polymer film having a thickness of 13 $\mu$m and a hot melt adhesive layer on a surface of a styrene elastomer having a sheet shape (manufactured by Kuraray Trading Co., Ltd.) was placed such that the hot melt adhesive layer was put on the surface of the elastomer, a surface opposite to an installation surface of the film was heated at 110°C, and was then further heated in a thermostatic chamber at 140°C for 10 minutes. As a result, a skin wearing member having a configuration in which the film was provided on the surface of the sheet-shaped elastomer was produced. In this skin wearing member, the sheet-shaped styrene elastomer corresponds to the base member containing the elastomer, and the film obtained by stacking the polyurethane-based polymer film and the hot melt adhesive layer corresponds to the film disposed on the surface of the base member. For this skin wearing member, Asker C hardness and a coefficient of static friction of a person were evaluated by the same method as in Example 1. As a result, the Asker C hardness was C6, and the coefficient of static friction for the artificial skin was 0.7.

(Evaluation of adhesion between base member and film in skin wearing member)

For the skin wearing member of Example 6 produced by the above method, an adhesive force between the sheet-shaped elastomer corresponding to the base member containing the elastomer and the film was evaluated by a peel test. As a result, the adhesive force was 1.48 N. In the peel test, according to the test method of ASTM F88, a maximum tensile strength obtained when the film was peeled off at a speed of 200 mm/min was measured by the force gauge systems ZTA-50N and MX2-500N-FA (manufactured by IMADA Co., Ltd.) by using a test piece having a width of 15 mm.
In order to evaluate the durability of the adhesion between the base member and the film in the use environment of the care device, a high temperature and high humidity test was performed. The skin wearing member of Example 6 was left for 1 week under a high temperature and high humidity test condition of 40°C and 95%. As a result, it was confirmed that the film surface was not peeled off after the test.

<Example 7>

(Preparation of skin wearing member)

A film (manufactured by Kuraray Trading Co., Ltd.) obtained by stacking a polyurethane-based polymer film having a thickness of 13 $\mu$m and a hot melt adhesive layer on a surface of a styrene elastomer having a sheet shape (manufactured by Kuraray Trading Co., Ltd.) was placed such that the hot melt adhesive layer was put on a surface of the elastomer, and an installation surface of the film was heated at 110°C. As a result, a skin wearing member having a configuration in which the film was provided on the surface of the sheet-shaped elastomer was produced. In this skin wearing member, the sheet-shaped styrene elastomer corresponds to a base member containing an elastomer, and a film obtained by stacking the polyurethane-based polymer film and the hot melt layer corresponds to the film disposed on the surface of the base member. For this skin wearing member, Asker C hardness and a coefficient of static friction of a person were evaluated by the same method as in Example 1. As a result, the Asker C hardness was C6, and the coefficient of static friction for the artificial skin was 1.3.

(Evaluation of adhesion between base member and film in skin wearing member)

The adhesion evaluation and the high temperature and high humidity test were performed on the skin wearing member of Example 7 produced by the above method by the same method as in Example 6. As a result, it was confirmed that an adhesive force of the film was 0.48 N and the film surface was not peeled off after the high temperature and high humidity test.

<Example 8>

(Preparation of skin wearing member)

A film (manufactured by Kuraray Trading Co., Ltd.) obtained by stacking a polyurethane-based polymer film having a thickness of 13 $\mu$m and a hot melt adhesive layer on a surface of a styrene elastomer having a sheet shape (manufactured by Kuraray Trading Co., Ltd.) was placed such that the hot melt adhesive layer was put on a surface of an elastomer, and a surface opposite to an installation surface of the film was heated at 110°C. As a result, a skin wearing member having a configuration in which the film was provided on the surface of the sheet-shaped elastomer was produced. In this skin wearing member, the sheet-shaped styrene elastomer corresponds to a base member containing an elastomer, and a film obtained by stacking the polyurethane-based polymer film and the hot melt layer corresponds to the film disposed on the surface of the base member. For this skin wearing member, Asker C hardness and a coefficient of static friction of a person were evaluated by the same method as in Example 1. As a result, the Asker C hardness was C6, and the coefficient of static friction for the artificial skin was 1.3.

(Evaluation of adhesion between base member and film in skin wearing member)

The adhesion evaluation and the high temperature and high humidity test were performed on the skin wearing member of Example 8 produced by the above method by the same method as in Example 6. As a result, it was confirmed that an adhesive force of the film was 0.34 N and the film surface was not peeled off after the high temperature and high humidity test.

<Example 9>

(Preparation of skin wearing member)

A film (manufactured by Kuraray Trading Co., Ltd.) obtained by stacking a polyurethane-based polymer film having a thickness of 13 $\mu$m and a hot melt adhesive layer on a surface of a styrene elastomer having a sheet shape (manufactured by Kuraray Trading Co., Ltd.) was placed such that the hot melt adhesive layer was put on a surface of an elastomer, and a surface opposite to an installation surface of the film was heated at 100°C. As a result, a skin wearing member having a configuration in which the film was provided on the surface of the sheet-shaped elastomer was produced. In this skin wearing member, the sheet-shaped styrene elastomer corresponds to a base member containing an elastomer, and a film obtained by stacking the polyurethane-based polymer film and the hot melt layer corresponds to the film disposed on the surface of the base member. For this skin wearing member, Asker C hardness and a coefficient of static friction of a person were evaluated by the same method as in Example 1. As a result, the Asker C hardness was C6, and the coefficient of static friction for the artificial skin was 1.3.

(Evaluation of adhesion between base member and film in skin wearing member)

The adhesion evaluation and the high temperature and high humidity test were performed on the skin wearing member of Example 9 produced by the above method by the same method as in Example 6. As a result, an adhesive force of the film was 0.22 N, and a part of the film was peeled off after the high temperature and high humidity test.

The results of Examples 6 to 9 are represented in Table 2. A case where the film was not peeled off after the high temperature and high humidity test was defined as A, and a case where the film was peeled off was defined as B. In Examples 6 to 8, the film after the high temperature and high humidity test was not peeled off, and in Example 9, a part of the film after the high temperature and high humidity test was peeled off. In a case where an adhesive force of the film by the peel test was more than or equal to 0.3 N, good results were obtained.

Table 2

| | Example 6 | Example 7 | Example 8 | Example 9 |
|---|---|---|---|---|
| Film adhesion (N) | 1.48 | 0.48 | 0.34 | 0.22 |
| High temperature and high humidity test | A | A | A | B |

<Example 10>

(Preparation of skin wearing member)

A styrene elastomer having a sheet shape (manufactured by TANAC Co., Ltd.) was prepared as a base member. A surface of the sheet was coated with powder containing talc as a main component (manufactured by WAKODO). As described above, the skin wearing member formed by applying the powder coating to the base member was prepared. In a skin wearing member, an Asker C hardness was 13 (C13), and a coefficient of static friction for the artificial skin was 1.4. This skin wearing member was used as a skin wearing member of Example 10.

(Tensile test)

Since a tensile force is applied to the skin wearing member when the care device is used, durability against repeated tensile forces is required. Accordingly, tensile strength evaluation and a tensile durability test were performed on the skin wearing member.
In the tensile strength test, a test piece having a width of 5 mm and a length of 30 mm was prepared, and stress at the time of breakage when the test piece was pulled at a tensile speed of 100 mm/min was calculated by using Autograph (EZ-L) manufactured by Shimadzu Corporation to obtain tensile strength. In the tensile durability test, a test piece having a thickness of 2 mm was used, a test piece having a width of 10 mm, a thickness of 2 mm, and a length of 30 mm was prepared, and when a pulling operation was repeated up to 300 times with a force of 2 N, the presence or absence of breakage up to 300 times was evaluated. It was confirmed that the tensile strength of the skin wearing member of Example 10 was 10.2 $N \cdot mm^2$ and there was no breakage until the tensile operation was performed 300 times.

<Example 11>

(Preparation of skin wearing member)

The same skin wearing member as in Example 1 was prepared.

(Tensile test)

Tensile strength evaluation and a tensile durability test were performed on a skin wearing member of Example 11 by the same method as in Example 10. The tensile strength of the skin wearing member of Example 11 was 3.1 $N \cdot mm^2$, and it was confirmed that there was no breakage until the tensile operation was performed 300 times.

<Example 12>

(Preparation of skin wearing member)

A silicone gel having a sheet shape (manufactured by Taica Corporation) was prepared as a base member. A surface of the sheet was coated with powder containing talc as a main component (manufactured by WAKODO). As described above, the skin wearing member formed by applying the powder coating to the base member was prepared. In the skin wearing member, an Asker C hardness was 0 (C0), and a coefficient of static friction for the artificial skin was 2.1. This skin wearing member was used as a skin wearing member of Example 12.

(Tensile test)

Tensile strength evaluation and a tensile durability test were performed on the skin wearing member of Example 12 in the same method as in Example 10. The tensile strength of the skin wearing member of Example 12 was 1.0 $N \cdot mm^2$, and the skin wearing member was broken within 300 times of the tensile operation.
The results of Examples 10 to 12 are represented in Table 3. A case where the film was not broken until the tensile operation was performed 300 times was defined as A, and a case where the film was broken within 300 times was defined as B. In Examples 10 and 11, the elastomer was not broken in the tensile durability test in 300 times of the tensile operation, and in Example 12, the elastomer was broken in 300 times or less of the tensile operation, and therefore the result of the tensile durability test was good for the elastomer having high tensile strength.

Table 3

|  | Example 10 | Example 11 | Example 12 |
|---|---|---|---|
| Elastomer material | Polystyrene | Silicone Gel | Silicone Gel |
| Tensile strength (N/mm$^2$) | 10.2 | 3.1 | 1.0 |
| Tensile durability test | A | A | B |

<Example 13>

(Preparation of skin wearing member)

Films (manufactured by Kuraray Trading Co., Ltd.) each obtained by stacking a polyurethane-based polymer film having a thickness of 13 $\mu$m and a hot melt adhesive layer on both surfaces of a styrene elastomer having a sheet shape (manufactured by Kuraray Trading Co., Ltd.) were installed such that the hot melt adhesive layer faced the elastomer, and end portions of two films adhered to each other with an adhesive layer to obtain a skin wearing member in which the film was installed on the entire surface of the sheet-shaped elastomer and sealed. In this skin wearing member, the sheet-shaped styrene elastomer corresponds to a base member containing an elastomer, and films each obtained by stacking the polyurethane-based polymer film and the hot melt layer corresponds to the film disposed on the surface of the base member. For this skin wearing member, Asker C hardness and a coefficient of static friction of a person were evaluated by the same method as in Example 1. As a result, the Asker C hardness was C6, and the coefficient of static friction for the artificial skin was 0.6.

<Oil resistance evaluation>

Since the skin wearing member is used while coming into contact with the skin, swelling and bleeding of an oil component on the skin or a cosmetic component applied to the skin are required to be small, and high oil resistance is required. As the oil resistance evaluation, from the viewpoint of the cosmetic component to be applied to the skin, silicone oil was employed. The skin wearing member was immersed in the silicone oil at 50°C for 3 days, and a mass change rate from before immersion to after immersion was evaluated. KF-96-100CS manufactured by Shin-Etsu Chemical Co., Ltd. was used as the silicone oil, and the mass measurement after immersion was performed after the oil adhering to the skin wearing member was wiped off. A mass of the skin wearing member of Example 13 after immersion was reduced by 1% as compared with a mass before immersion.

<Example 14>

(Preparation of skin wearing member)

The same skin wearing member as in Example 3 was prepared.

<Oil resistance evaluation>

As a result of evaluating the oil resistance of the skin wearing member of Example 14 by the same method as in Example 13, the mass after immersion in silicone oil was reduced by 49% as compared with the mass before immersion.

<Example 15>

(Preparation of skin wearing member)

The same skin wearing member as in Example 2 was prepared.

<Oil resistance evaluation>

As a result of evaluating the oil resistance of the skin wearing member of Example 15 by the same method as in Example 13, the mass after immersion in silicone oil was reduced by 80% as compared with the mass before immersion.

The results of Examples 13 to 15 are represented in Table 4. A case where a mass change before and after silicone oil immersion was less than or equal to 10% was defined as oil resistance A, a case where the mass change before and after silicone oil immersion was less than or equal to 50% was defined as oil resistance B, and a case where the mass change before and after silicone oil immersion was more than 50% was defined as C. The oil resistance was improved by installing the films on the entire surface of the base member containing the elastomer and bonding and sealing the end portions of the films.

Table 4

|  | Example 13 | Example 14 | Example 15 |
|---|---|---|---|
| Film installation | Entire surface (sealed with film) | Both surfaces | None |
| Oil resistance evaluation | A | B | C |

<Example 16>

(Pushing depth evaluation of skin wearing member)

The skin wearing member was cut into a horizontal width of 20 cm and a vertical width of 7 cm by using the skin wearing member used in Example 6. As illustrated in Fig. 1, a care device was produced by coupling the skin wearing member at supports at both ends in a horizontal width direction of a frame having a horizontal width of 20 cm and a horizontal width of 7 cm and a depth of 5 cm. A pushing depth in a direction perpendicular to a surface of a skin contact surface of the skin wearing member of this care device when a load of 1 N was applied in the direction perpendicular was measured and found to be 23 mm. The pushing depth was evaluated by measuring displacement of the skin wearing member in the perpendicular direction when a load was applied by using a cylindrical pushing jig having a diameter of 4 mm by using force gauge systems ZTA-50N and MX2-500N-FA (manufactured by IMADA Co., Ltd.).

(Pleasantness evaluation)

The pleasantness of the touch feeling of the eye when the care device produced in Example 16 was worn on the eye in a state where the skin contact surface of the skin wearing member was brought into contact with the skin was evaluated. Subjects were five men and women in their 40s to 50s, and the scores were kept based on the following criteria, and average values of the scores of the subjects were calculated.

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

The pleasantness of the touch feeling of the eye was 6.3 points.

<Example 17>

(Pushing depth evaluation of skin wearing member)

The skin wearing member was cut into a horizontal width of 20 cm and a vertical width of 7 cm by using the skin wearing member used in Example 6. As illustrated in Fig. 4, a care device in which the skin wearing member was coupled at supports at both ends in a horizontal width direction of a frame having a horizontal width of 20 cm, a horizontal width of 7 cm, and a depth of 5 cm and a silicone rubber sheet A50 (manufactured by TOGAWA RUBBER CO., LTD.) having a thickness of 1 mm was installed as an underlying member at a position opposite to a skin wearing surface of the skin wearing member was produced. A pushing depth of the care device of Example 17 thus obtained was evaluated by the same method as in Example 16. As a result, the pushing depth was 16.3 mm.

(Pleasantness evaluation)

When the pleasantness of the care device produced in Example 17 was evaluated by the same method as in Example 16, the pleasantness was 6.0.

<Example 18>

(Pushing depth evaluation of skin wearing member)

The skin wearing member was cut into a horizontal width of 20 cm and a vertical width of 7 cm by using the skin wearing member used in Example 6. As illustrated in Fig. 4, a care device in which the skin wearing member was coupled at supports at both ends in a horizontal width direction of a frame having a horizontal width of 20 cm, a horizontal width of 7 cm, and a depth of 5 cm and a urethane sponge (manufactured by TRUSCO NAKAYAMA CORPORATION) was installed as an underlying member at a position opposite to a skin wearing surface of the skin wearing member was produced. A pushing depth of the care device of Example 17 thus obtained was evaluated by the same method as in Example 16, and the pushing depth was 5.0 mm.

(Pleasantness evaluation)

When the pleasantness of the care device produced in Example 18 was evaluated by the same method as in Example 16, the pleasantness was 6.3.

<Example 19>

(Pushing depth evaluation of skin wearing member)

The skin wearing member was cut into a horizontal width of 20 cm and a vertical width of 7 cm by using the skin wearing member used in Example 6. As illustrated in Fig. 4, a care device in which the skin wearing member was coupled at supports at both ends in a horizontal width direction of a frame having a horizontal width of 20 cm, a horizontal width of 7 cm, and a depth of 5 cm, and a silicone rubber sheet A50 (manufactured by TOGAWA RUBBER CO., LTD.) having a thickness of 2 mm as an underlying member and a urethane sponge (manufactured by TRUSCO NAKAYAMA CORPORATION) having a thickness of 10 mm were installed in an overlapping manner at a position opposite to a skin wearing surface of the skin wearing member was produced. A pushing depth of the care device of Example 19 thus obtained was evaluated by the same method as in Example 16. As a result, the pushing depth was 1.0 mm.

(Pleasantness evaluation)

The pleasantness of the care device produced in Example 19 was evaluated by the same method as in Example 16. As a result, the pleasantness was 4.1.

<Example 20>

(Pushing depth evaluation of skin wearing member)

The skin wearing member was cut into a horizontal width of 18 cm and a vertical width of 6 cm by using the skin wearing member used in Example 6. A care device was produced by attaching the skin wearing member to an eye care device (eye treatment manufactured by Panasonic Corporation) used as a frame with a skin contact surface of the eye care device as a support. The pushing depth of the care device of Example 20 thus obtained was evaluated by the same method as in Example 16. As a result, the pushing depth was 0.8 mm.

(Pleasantness evaluation)

The pleasantness of the care device produced in Example 20 was evaluated by the same method as in Example 16. As a result, the pleasant was 4.0.
The results of Examples 16 to 20 are represented in Table 5. In Examples 16 to 19, the score of the pleasantness of the touch feeling of the eye of the care device was higher than 4 points, and in Example 20, the score of the pleasantness of the touch feeling was less than or equal to 4 points. In a case where the skin wearing member containing the elastomer was coupled to at least a part of the frame and the pushing depth in the perpendicular direction when a load of 1 N was applied to the surface of the skin contact surface in the perpendicular direction was more than or equal to 1 mm, the result in which the pleasantness when the care device was worn was high was obtained.

Table 5

| | Example 16 | Example 17 | Example 18 | Example 19 | Example 20 |
|---|---|---|---|---|---|
| Pushing depth of skin wearing member (mm) | 23.0 | 16.3 | 5.0 | 1.0 | 0.8 |
| Pleasantness of touch feeling of eye | 6.3 | 6.0 | 6.3 | 4.1 | 4.0 |

<Example 21>

The skin wearing member was cut into a horizontal width of 20 cm and a vertical width of 7 cm by using the skin wearing member used in Example 6. As illustrated in Fig. 4, a care device in which the skin wearing member was coupled at supports at both ends in a horizontal width direction of a frame having a horizontal width of 20 cm, a horizontal width of 7 cm, and a depth of 5 cm, and a urethane sponge (manufactured by TRUSCO NAKAYAMA CORPORATION) having a thickness of 10 mm as an underlying member was installed in an overlapping manner at a position opposite to a skin wearing surface of the skin wearing member was produced.

(Evaluation of compressive stress of underlying member)

A 10% compressive stress of the urethane sponge as the underlying member used in Example 21 was measured and found to be 0.004 MPa. The 10% compressive stress was obtained as follows. A surface of a sample having a prismatic shape (here, the urethane sponge used as the underlying member) adjusted to a width of 10 mm, a length of 10 mm, and a thickness of 5 mm was compressed at a speed of 10 mm/min by using force gauge systems ZTA-50N and MX2-500N-FA (manufactured by IMADA Co., Ltd.) and using a probe having a cylindrical shape and having a diameter of 40 mm was as a probe. A compressive force when the sample was 10% compressed from an original thickness was measured. Compressive stress σ was calculated by the following Equation (2) from compressive force F as a measurement result and area A of a surface to which the load was applied, which was a sectional area of the sample before the load was applied.

$$\sigma = F/A \text{ (Equation 2)}$$

(Pleasantness evaluation)

The pleasantness of the touch feeling of the eye when the care device produced in Example 21 was worn on the eye in a state where the skin contact surface of the skin wearing member was brought into contact with the skin was evaluated. Subjects were five men and women in their 40s to 50s, and the scores were kept based on the following criteria, and average values of the scores of the subjects were calculated.

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

The pleasantness of the touch feeling of the eye of the care device of Example 21 was 6.8 points.

<Example 22>

A care device was produced by the same method as in Example 21 except that foamed polyethylene (manufactured by TRUSCO NAKAYAMA CORPORATION) having a thickness of 5 mm was used as an underlying member. For this care device, a 10% compressive stress of the underlying member was evaluated by the same method as in Example 21. As a result, the 10% compressive stress was 0.01 MPa. When the pleasantness of the touch feeling of the eye of the care device was evaluated by the same method as in Example 21, the pleasantness of the touch feeling of the eye was 5.0 points.

<Example 23>

A care device was produced by the same method as in Example 21 except that silicone rubber A50 (manufactured by TOGAWA RUBBER CO., LTD.) having a thickness of 5 mm was used as an underlying member. For this care device, a 10% compressive stress of the underlying member was evaluated by the same method as in Example 21. As a result, the 10% compressive stress was 0.14 MPa. When the pleasantness of the touch feeling of the eye of the care device was evaluated by the same method as in Example 21, the pleasantness of the touch feeling of the eye was 4.5 points.

<Example 24>

A care device was produced by the same method as in Example 21 except that a urethane rubber 90° (manufactured by KOKUGO Co., Ltd.) having a thickness of 3 mm was used as an underlying member. For this care device, a 10% compressive stress of the underlying member was evaluated by the same method as in Example 21. As a result, the 10% compressive stress was 0.28 MPa. When the pleasantness of the touch feeling of the eye of the care device was evaluated by the same method as in Example 21, the pleasantness of the touch feeling of the eye was 4.0 points.

The results of Examples 21 to 24 are represented in Table 6. In Examples 21 to 23, the score of the pleasantness of the touch feeling of the eye of the care device was higher than 4 points, and in Example 24, the pleasantness was less than or equal to 4. In a case where the underlying member was provided at a position opposite to a skin wearing surface of the skin wearing member, and the 10% compressive stress of the underlying member was less than or equal to 0.2 MPa, the result in which the pleasantness when the care device was worn was high was obtained.

Table 6

|  | Example 21 | Example 22 | Example 23 | Example 24 |
|---|---|---|---|---|
| 10% compressive stress of underlying member (MPa) | 0.004 | 0.013 | 0.14 | 0.28 |
| Pleasantness of touch feeling of eye | 6.8 | 5.0 | 4.5 | 4.0 |

<Example 25>

The skin wearing member was cut into a horizontal width of 50 mm and a vertical width of 100 mm by using the skin wearing member used in Example 6. A care device in which a frame having a horizontal width of 50 mm and a vertical width of 100 mm was installed at a position opposite to a skin wearing surface of the skin wearing member was produced. An area of the skin wearing surface of the skin wearing member of the care device of Example 25 was 50 cm$^2$.

(Pleasantness evaluation)

The pleasantness of the touch feeling when the care device produced in Example 25 was worn on an upper arm in a state where a skin wearing surface of the skin wearing member was brought into contact with the skin was evaluated. Subjects were five men and women in their 30s to 50s, and the scores were kept based on the following criteria, and average values of the scores of the subjects were calculated.

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

The pleasantness of the touch feeling of the care device of Example 25 was 7.0 points.

<Example 26>

A care device was produced by the same method as in Example 25 except that the skin wearing member was cut into a horizontal width of 50 mm and a vertical width of 50 mm and a frame having a horizontal width of 50 mm and a vertical width of 50 mm was installed at a position opposite to a skin wearing surface of the skin wearing member, and the pleasantness of the touch feeling when the care device was worn on an upper arm was evaluated. The pleasantness of the touch feeling of the care device of Example 26 was 5.8. An area of the skin contact surface of the skin wearing member of the care device of Example 26 was 25 cm$^2$.

<Example 27>

A care device was produced by the same method as in Example 25 except that the skin wearing member was cut into a horizontal width of 30 mm and a vertical width of 50 mm and a frame having a horizontal width of 30 mm and a vertical width of 50 mm was installed at a position opposite to a skin wearing surface of the skin wearing member, and the pleasantness of the touch feeling when the care device was worn on an upper arm was evaluated. The pleasantness of the touch feeling of the care device of Example 27 was 5.3. An area of the skin contact surface of the skin wearing member of the care device of Example 27 was 15 cm$^2$.

<Example 28>

A care device was produced by the same method as in Example 25 except that the skin wearing member was cut into a horizontal width of 30 mm and a vertical width of 30 mm and a frame having a horizontal width of 30 mm and a vertical width of 30 mm was installed at a position opposite to a skin wearing surface of the skin wearing member, and the pleasantness of the touch feeling when the care device was worn on an upper arm was evaluated. The pleasantness of the touch feeling of the care device of Example 28 was 4.8. An area of the skin contact surface of the skin wearing member of the care device of Example 28 was 9 cm$^2$.

<Example 29>

A care device was produced by the same method as in Example 25 except that the skin wearing member was cut into a horizontal width of 20 mm and a vertical width of 20 mm and a frame having a horizontal width of 20 mm and a vertical width of 20 mm was installed at a position opposite to a skin wearing surface of the skin wearing member, and the pleasantness of the touch feeling when the care device was worn on an upper arm was evaluated. The pleasantness of the touch feeling of the care device of Example 29 was 4.0. An area of the skin contact surface of the skin wearing member of the care device of Example 29 was 4 cm$^2$.

The results of Examples 25 to 29 are represented in Table 7. In a case where the score of the pleasantness of the touch feeling when the care device was worn was higher than 4 points in Examples 25 to 28, the score of the pleasantness was less than or equal to 4 in Example 24, and the area of the skin wearing surface of the skin wearing member was more than 4 cm$^2$, the result in which the pleasantness when the care device was worn was high was obtained.

Table 7

|  | Example 25 | Example 26 | Example 27 | Example 28 | Example 29 |
|---|---|---|---|---|---|
| Area of skin contact surface of skin wearing member (cm$^2$) | 50 | 25 | 15 | 9 | 4 |
| Pleasantness of touch feeling when care device was worn | 7.0 | 5.8 | 5.3 | 4.8 | 4.0 |

<Example 30>

A care device including a skin wearing member, a frame, a heating element, and a vibrator as illustrated in Fig. 7 was produced. The care device was produced by using the skin wearing member used in Example 3 as the skin wearing member and coupling the skin wearing member to the frame by a support. The care device was worn on the eye, the skin wearing member was warmed by the heating element, care for vibrating the skin wearing member was performed by the vibrator, and the pleasantness when the care device was worn, the pleasantness during the care, the relaxation feeling, and the soothing and healing feeling were evaluated. Subjects were 11 men and women in their 20s to 50s, sat on a reclining seat, wore the care device by fixing a rubber band coupled to a frame to a head, performed caring for 3 minutes.

Thereafter, the subjects kept scores based on the following criteria, and average values of the scores of the subjects were calculated.

<Pleasantness when care device was worn>

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

<Pleasantness during care>

Score 7: extremely pleasant
Score 6: very pleasant
Score 5: slightly pleasant
Score 4: neither pleasant nor unpleasant
Score 3: slightly unpleasant
Score 2: very unpleasant
Score 1: extremely unpleasant

<Relaxation feeling>

Score 7: extremely relaxing
Score 6: very relaxing
Score 5: slightly relaxing
Score 4: neither relaxing nor unrelaxing
Score 3: slightly unrelaxing
Score 2: very unrelaxing
Score 1: extremely unrelaxing

<Soothing and healing>

Score 7: extremely soothing and healing
Score 6: very soothing and healing
Score 5: slightly soothing and healing
Score 4: neither soothing/healing nor non-soothing/non-healing
Score 3: slightly non-soothing and non-healing
Score 2: very non-soothing and non-healing
Score 1: extremely non-soothing and non-healing

<Example 31>

A care device including a skin wearing member, a frame, and a heating element as illustrated in Fig. 5 was produced. The care device was produced by using the skin wearing member used in Example 6 as the skin wearing member and coupling the skin wearing member to the frame by a support. The care device was worn on the eye, the skin wearing member was warmed by the heating element, and the pleasantness when the care device was worn, the pleasantness during the care, the relaxation feeling, and the soothing and healing feeling were evaluated by the same method as in Example 30.

<Comparative Example 4>

An eye care device (manufactured by Panasonic Corporation) in which silicone rubber having an Asker C hardness of 70 (C70) and a coefficient of static friction for the artificial skin of 1.4 was installed was used as a skin wearing member. The pleasantness when the care device was worn, the pleasantness during the care, the relaxation feeling, and the smoothing and healing feeling were evaluated by the same method as in Example 30 except that the skin wearing member was warmed by the heating element included in the eye care device and care for vibrating the skin wearing member by the vibrator was performed. Setting conditions of the care device were a high temperature and a quick mode.

The results of Example 30, Example 31, and Comparative Example 4 are represented in Table 8. In Example 30 and Example 31 compared to Comparative Example 4, the pleasantness when the care device was worn, the pleasantness during the care, the relaxation feeling, the soothing feeling, and the healing feeling were high.

Table 8

|  | Example 30 | Example 31 | Comparative Example 4 |
|---|---|---|---|
| Pleasantness when care device was worn | 5.8 | 5.6 | 3.8 |
| Pleasantness during care | 5.8 | 5.6 | 4.5 |
| Relaxation feeling | 5.9 | 5.9 | 4.3 |
| Soothing and healing feeling | 6.1 | 5.5 | 4.3 |

<Example 32>

(Vibration feeling evaluation)

A care device including a skin wearing member, a frame, a heating element, and a vibrator as illustrated in Fig. 7 was produced. The care device was produced by using the skin wearing member used in Example 6 as the skin wearing member and coupling the skin wearing member to the frame by a support. As illustrated in Fig. 8, silicone rubber A50 (manufactured by TOGAWA RUBBER CO., LTD.) having a thickness of 2 mm and a size of 10 mm × 80 mm was used as a vibration assist jig and was installed on a surface opposite to the skin contact surface of the skin wearing member to be positioned at a lower portion of the eye. The vibrator was installed to be positioned outside the eye. When a mass per unit area of the installed vibration assist jig was evaluated, the mass per unit area was 0.27 g/cm$^2$. The care device was worn on the eye, care for vibrating the skin wearing member by the vibrator was performed, and the vibration feeling under the eye as a jig installation portion was evaluated for the vibration feeling felt by the skin. A rubber band coupled to the frame was fixed to the head to wear the care device, care was performed for 3 minutes, and then evaluation was performed based on the following criteria.

(Vibration feeling)

A: vibration feeling under eye was stronger than other portions of skin wearing member
B: vibration feeling under eye was equivalent to other portions of skin wearing member
C: vibration feeling under eye was less than other portions of skin wearing member

<Example 33>

A care device was produced by the same method as in Example 32 except that a stainless steel ball having Φ10 mm was used as a vibration assist jig instead of the silicone rubber A50 having the thickness of 2 mm and the size of 10 mm × 80 mm. A mass per unit area of the installed jig was evaluated. As a result, the mass was 4.2 g/cm$^2$. This care device was worn on the eye, and the vibration feeling was evaluated by the same method as in Example 32.

<Example 34>

A care device was produced by the same method as in Example 32 except that a silicone gel (manufactured by TANAC Co., Ltd.) having a thickness of 10 mm and a size of 10 mm × 80 mm was used as a vibration assist jig instead of the silicone rubber A50 having the thickness of 2 mm and the size of 10 mm × 80 mm. A mass per unit area of the installed jig was evaluated, and as a result, the mass was 1.4 g/cm². This care device was worn on the eye, and the vibration feeling was evaluated by the same method as in Example 32.

<Example 35>

A care device was produced by the same method as in Example 32 except that a PET film (manufactured by TORAY INDUSTRIES, INC.) having a thickness of 200 μm and a size of 10 mm × 80 mm was used as a vibration assist jig instead of the silicone rubber A50 having the thickness of 2 mm and the size of 10 mm × 80 mm. A mass per unit area of the installed jig was evaluated. As a result, the mass was 0.03 g/cm². This care device was worn on the eye, and the vibration feeling was evaluated by the same method as in Example 32.

<Example 36>

A care device was produced by the same method as in Example 32 except that foamed polyethylene (manufactured by TRUSCO NAKAYAMA CORPORATION) having a thickness of 5 mm and a size of 10 mm × 80 mm was used as a vibration assist jig instead of the silicone rubber A50 having the thickness of 2 mm and the size of 10 mm × 80 mm. A mass per unit area of the installed jig was evaluated. As a result, the mass was 0.015 g/cm². This care device was worn on the eye, and the vibration feeling was evaluated by the same method as in Example 23.

<Example 37>

A care device was produced by the same method as in Example 32 except that a polyimide tape having a thickness of 100 μm and a size of 10 mm × 80 mm was used as a vibration assist jig instead of the silicone rubber A50 having the thickness of 2 mm and the size of 10 mm × 80 mm. A mass per unit area of the installed jig was evaluated. As a result, the mass was 0.015 g/cm². This care device was worn on the eye, and the vibration feeling was evaluated by the same method as in Example 32.

The results of Examples 32 to 37 are represented in Table 9. In Examples 32 to 35 in which the mass per unit area of the vibration assist jig was more than or equal to 0.02 g/cm², the vibration feeling under the eye as the vibration assist jig installation portion was more than or equal to the vibration feeling of the other portion where the jig was not installed. In Examples 36 and 37 in which the mass per unit area of the vibration assist jig was less than 0.02 g/cm², the vibration feeling under the eye as the vibration assist jig installation portion was less than the other portions of the skin wearing member in which the vibration assist jig was not installed. As described above, the vibration assist jig made of a solid object having a mass per unit area of 0.02 g/cm² or more on the surface opposite to the skin contact surface of the skin wearing member was installed, and thus, the vibration feeling in the vibration assist jig installation portion of the skin wearing member was improved.

Table 9

|  | Example 32 | Example 33 | Example 34 | Example 35 | Example 36 | Example 37 |
|---|---|---|---|---|---|---|
| Mass of vibration assist jig per unit area (g/cm²) | 0.27 | 4.2 | 1.4 | 0.03 | 0.015 | 0.015 |
| Vibration feeling | A | A | A | B | C | C |

INDUSTRIAL APPLICABILITY

The care device that is brought into contact with or worn on the skin according to the present disclosure can be used for applications such as a care device that massages an eye, a face, a neck, a hand, a foot, or the like, obtains a cosmetic effect such as a beauty treatment, or obtains an effect such as relaxation, healing, or sleep introduction.

REFERENCE MARKS IN THE DRAWINGS

1: skin wearing member
2: skin contact surface

3: frame
4: support
5: underlying member
6: heating element
7: vibrator
8: vibration assist jig
11: base member
12, 13: film
14: adhesive layer
100, 200, 300, 400, 500, 600: care device

**Claims**

1. A care device that is brought into contact with or worn on a skin, the care device comprising:

   a skin wearing member containing an elastomer,
   wherein
   the skin wearing member has an Asker C hardness of 30 degrees or less and a coefficient of static friction of 3 or less,
   where
   the coefficient of static friction is a coefficient of static friction for an artificial skin sheet that satisfies following characteristics (A) and (B):

   (A) a compressive stress of the artificial skin sheet at a time of being pushed in a thickness direction at a depth of 2 mm is between 0.01 MPa and 0.05 MPa inclusive; and
   (B) calculated average roughness Ra of a surface of the artificial skin sheet is from 5 $\mu$m to 10 $\mu$m.

2. The care device according to Claim 1, wherein
   a tensile strength of the skin wearing member is more than 1 N/mm$^2$.

3. The care device according to Claim 1, wherein
   the skin wearing member includes:

   a base member containing the elastomer, and
   a film having a thickness of 100 $\mu$m or less, the film being disposed in at least a region of a surface of the base member facing the skin.

4. The care device according to Claim 3, wherein
   an adhesive force between the base member and the film is more than or equal to 0.3 N in a peel test at a width of 15 mm.

5. The care device according to Claim 3, wherein
   the film covers an entire surface of the base member.

6. The care device according to Claim 1, wherein
   the skin wearing member includes:

   a base member containing the elastomer, and
   a coating containing powder disposed in at least a region of a surface of the base member facing the skin.

7. The care device according to Claim 1, wherein
   a pushing depth in a direction perpendicular to a surface of a portion of the skin wearing member coming into contact with the skin when a load of 1 N is applied to the surface in a perpendicular direction by using a pushing jig having a cylindrical shape and having a diameter of 5 mm is more than or equal to 1 mm.

8. The care device according to Claim 1, further comprising:

an underlying member,
wherein
the underlying member is provided at a position opposite to the skin with respect to the skin wearing member and comes into contact with at least a part of a portion of the skin wearing member not coming into contact with the skin in a state where the skin wearing member is worn on the skin, and
a 10% compressive stress of the underlying member is less than or equal to 0.2 MPa.

9. The care device according to Claim 1, wherein
an area of a skin wearing surface of the skin wearing member is more than 4 cm$^2$.

10. The care device according to Claim 1, wherein
the elastomer is a gel containing polystyrene as a main material, a gel containing silicone as the main material, or silicone rubber.

11. The care device according to Claim 1, wherein
the skin wearing member is made of a polymer material containing the elastomer.

12. The care device according to Claim 1, further comprising:
a heating element configured to continuously control a temperature.

13. The care device according to Claim 1, further comprising:
a cooling controller that cools the skin wearing member to control a temperature.

14. The care device according to Claim 1, further comprising:
a vibrator that vibrates the skin wearing member.

15. The care device according to Claim 1, further comprising:

a vibration assist jig,
wherein
the vibration assist jig is provided at a portion of the skin wearing member not coming into contact with the skin in a state where the skin wearing member is worn on the skin, and
the vibration assist jig is made of a solid material having a mass per unit area of 0.02 g/cm$^2$ or more and has a mass of 500 g or less.

16. The care device according to Claim 1, further comprising:
a pressure change unit that changes a pressure applied to the skin wearing member.

17. The care device according to Claim 1, further comprising:
a tensile force change unit that changes a tensile force applied to the skin wearing member.

18. The care device according to Claim 1, further comprising:

a skin portion detection unit that detects a portion of the skin with or on which the care device is brought into contact or worn; and
a temperature controller that heats or cools the skin wearing member to control a temperature in accordance with a result detected by the skin portion detection unit.

19. The care device according to Claim 14, further comprising:

a skin portion detection unit that detects a portion of the skin with or on which the care device is brought into contact or worn; and
a system that changes a vibration amount of the vibrator in accordance with a result detected by the skin portion detection unit.

20. The care device according to Claim 16, further comprising a skin portion detection unit that detects a portion of the skin with or on which the care device is brought into contact or is worn,
wherein

the pressure change unit changes a pressure applied to the skin wearing member in accordance with a result detected by the skin portion detection unit.

21. The care device according to Claim 17, further comprising a skin portion detection unit that detects a portion of the skin with or on which the care device is brought into contact or is worn,
wherein
the tensile force change unit changes the tensile force applied to the skin wearing member in accordance with a result detected by the skin portion detection unit.

22. The care device according to Claim 1, wherein
the care device is brought into contact with or worn on a face or a skin around a neck.

23. The care device according to Claim 1, wherein
the care device is brought into contact with or worn on a skin around an eye.

# FIG. 1

100

# FIG. 2

# FIG. 3A

# FIG. 3B

# FIG. 4

200

# FIG. 5

300

# FIG. 6

400

# FIG. 7

500

# FIG. 8

# FIG. 9

<u>600</u>

# FIG. 10

# FIG. 11

## INTERNATIONAL SEARCH REPORT

International application No.

**PCT/JP2023/039025**

| A. | CLASSIFICATION OF SUBJECT MATTER |
|---|---|

*A61H 7/00*(2006.01)i; *A61H 23/00*(2006.01)i; *A61F 7/00*(2006.01)i
FI: A61H7/00; A61H23/00; A61F7/00

According to International Patent Classification (IPC) or to both national classification and IPC

| B. | FIELDS SEARCHED |
|---|---|

Minimum documentation searched (classification system followed by classification symbols)

A61H7/00; A61H23/00; A61F7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2006-312035 A (SEIREN CO LTD) 16 November 2006 (2006-11-16)<br>paragraphs [0011], [0033], [0046]-[0047] | 1-23 |
| A | US 2012/0253248 A1 (CARLSON, Thomas G.) 04 October 2012 (2012-10-04)<br>paragraphs [0047], [0051] | 1-23 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **20 November 2023** | **05 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/039025**

| Patent document cited in search report | | | Publication date (day/month/year) | Patent family member(s) | | | Publication date (day/month/year) |
|---|---|---|---|---|---|---|---|
| JP | 2006-312035 | A | 16 November 2006 | CN | 1843321 | A | |
| US | 2012/0253248 | A1 | 04 October 2012 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0716255 B
- WO 2014119025 A
- JP 2004194734 A

**Non-patent literature cited in the description**

- **KOJI MIZUKOSHI et al.** Age-Related Changes of the Three-Dimensional Structure in the Cheek Region. *Journal of Society of Cosmetic Chemists of Japan*, 2009, vol. 43 (3), 177-183